# EUROPEAN PATENT SPECIFICATION

(11) **EP 3 551 644 B1**
(45) Date of publication and mention of the grant of the patent: **16.08.2023**
(21) Application number: 17828782.7
(22) Date of filing: 08.12.2017
(51) Int. Cl.: C07K 14/005, A61K 39/205, C12N 15/86

(54) **CHIMPANZEE ADENOVIRUS CONSTRUCTS WITH LYSSAVIRUS ANTIGENS**
SCHIMPANSEN-ADENOVIRUS-KONSTRUKTE MIT LYSSAVIRUS-ANTIGENEN
CONSTRUCTIONS D'ADÉNOVIRUS DE CHIMPANZÉ AVEC DES ANTIGÈNES DE LYSSAVIRUS

(30) Priority: 09.12.2016 US 201662432033 P; 01.03.2017 US 201762465378 P
(43) Date of publication of application: 16.10.2019
(73) Proprietor: GlaxoSmithKline Biologicals SA, 1330 Rixensart (BE)
(72) Inventor: AMMENDOLA, Virginia, 00128 Rome (IT); COLLOCA, Stefano, 00198 Rome (IT); VITELLI, Alessandra, 00128 Rome (IT); WIZEL, Benjamin, Rockville, Maryland 20850 (US)
(74) Representative: Hitchcock, Lucy Rose
(86) International application number: PCT/IB2017/057759
(87) International publication number: WO 2018/104919

(56) References cited:
- WO-A1-03/000283
- WO-A1-2015/189425
- WO-A1-2016/198599
- WO-A2-2005/071093
- WO-A2-2009/105084
- WO-A2-2010/086189
- XIANG Z Q ET AL: "Protection of non-human primates against rabies with an adenovirus recombinant vaccine", VIROLOGY, vol. 450, 9 January 2014 (2014-01-09), pages 243-249, XP028607745, ISSN: 0042-6822, DOI: 10.1016/J.VIROL.2013.12.029
- HILDEGUND C. J. ERTL ET AL: "Novel Vaccines to Human Rabies", PLOS NEGLECTED TROPICAL DISEASES, vol. 3, no. 9, 29 September 2009 (2009-09-29), page e515, XP055450808, US ISSN: 1935-2727, DOI: 10.1371/journal.pntd.0000515
- COLLOCA STEFANO ET AL: "Vaccine Vectors Derived from a Large Collection of Simian Adenoviruses Induce Potent Cellular Immunity Across Multiple Species", SCIENCE TRANSLATIONAL MEDICINE, AMERICAN ASSOCIATION FOR THE ADVANCEMENT OF SCIENCE (A A A S), US, vol. 4, no. 115, 1 January 2012 (2012-01-01), pages 47-55, XP009166675, ISSN: 1946-6234, DOI: 10.1126/SCITRANSLMED.3002925 cited in the application
- ASTRAY RENATO MANCINI ET AL: "Rabies vaccine development by expression of recombinant viral glycoprotein", ARCHIVES OF VIROLOGY, SPRINGER WIEN, AT, vol. 162, no. 2, 31 October 2016 (2016-10-31), pages 323-332, XP036155759, ISSN: 0304-8608, DOI: 10.1007/S00705-016-3128-9 [retrieved on 2016-10-31]
- DATABASE Geneseq [Online] 24 September 2015 (2015-09-24), "Rabies virus G protein, SEQ ID 22.", XP002778171, retrieved from EBI accession no. GSP:BCD43420 Database accession no. BCD43420 & WO 2015/119291 A1 (INST OF BIOLOG RESOURCES [JP]) 13 August 2015 (2015-08-13)

## Description

### FIELD OF THE INVENTION

This invention is in the field of ameliorating disease and treating and preventing viral infections. In particular, the present invention relates to chimpanzee adenoviral vectors encoding a *Lyssavirus* antigen. It includes the use of *Lyssavirus* antigens for ameliorating *Lyssaviral* diseases and treating and preventing rabies infections.

### BACKGROUND

*Lyssavirus* is an enveloped, single stranded RNA virus in the *Rhabdoviridae* family. Members of the *Lyssavirus* genus cause rabies and have the highest fatality rate of all known human viral pathogens. Rabies is transmitted via the saliva of infected mammals. A neurotropic virus, it enters the nervous system of its host, causing an encephalomyelitis that is almost invariably fatal. Currently there are about 60,000 rabies deaths worldwide yearly, mostly caused by dog bites in developing countries in Asia and Africa and by wildlife and bats in North America.

Rabies presents either in a furious or a paralytic form. The incubation period varies between about five days and several years but is typically between about 20 and 90 days. Clinical illness most often starts with prodromal complaints of malaise, anorexia, fatigue, headache and fever followed by pain or parathesia at the site of exposure. Anxiety, agitation or irritability may be prominent during this period, followed by hyperactivity, disorientation, seizures, hydrophobia, hypersalivation and, eventually, paralysis, coma and death.

Adenovirus has been widely used for gene transfer applications due to its ability to achieve highly efficient gene transfer in a variety of target tissues and large transgene capacity. Conventionally, adenovirus E1 genes are deleted and replaced with a transgene cassette consisting of a promoter of choice, cDNA sequence of the gene of interest and a poly A signal, resulting in a replication defective recombinant virus.

Recombinant adenoviruses are useful in both gene therapy and as vaccines. Viral vectors based on non-human simian adenovirus represent an alternative to the use of human derived vectors for the development of genetic vaccines. Certain adenoviruses isolated from non-human simians are closely related to adenoviruses isolated from humans, as demonstrated by their efficient propagation in cells of human origin.

There is a demand for vectors that can effectively deliver vaccine antigens. Specifically, rabies remains an important viral zoonosis worldwide. While prophylaxis is currently available, high numbers of doses are required both pre and post exposure, and compliance is low, diminishing the medical benefit. There is a need for an improved rabies vaccine with a simplified dosing schedule, increased safety and an enhanced manufacturing profile. Adenovirus manufacturing is safer and less expensive than the existing human rabies vaccines, which are based on inactivated rabies virus. Accordingly, there is an unmet need to develop adenoviral vectors for use in a rabies vaccine.

WO2015/189425A1 discloses immunogenic combinations that include a) an immunogenic component containing a peptide or polypeptide antigen of a respiratory pathogen; and b) an immunogenic component containing a nucleic acid encoding an antigen of the same respiratory pathogen, wherein the immunogenic components are formulated for concurrent, e.g., co-localized, administration.

WO2009/105084A2 discloses a recombinant vector comprises simian adenovirus SAdV-40, SAdV-31 or SAdV-34 sequences and a heterologous gene under the control of regulatory sequences.

WO2005/071093A2 discloses recombinant replication-defective adenoviral vectors derived from chimpanzee adenoviruses and methods for generating recombinant adenoviruses in human E1-expressing cell lines.

WO2010/086189A2 discloses novel adenovirus strains with an improved seroprevalence WO03/000283A1 discloses a method of inducing a CD8+ T response against a selected molecule by delivering the molecule via a recombinant simian adenovirus.

Xiang, Z. Q., et al. Virology 450 (2014): 243-249 discloses immunisation with a dose of a chimpanzee adenovirus vector SAd-V24 expressing rabies virus glycoprotein. Ertl, Hildegund CJ. PLoS neglected tropical diseases 3.9 (2009): e515 discloses novel vaccines to human rabies.

### SUMMARY OF THE INVENTION

The present inventors provide constructs useful as components of immunogenic compositions for the induction of an immune response in a subject against Lyssaviral diseases and rabies viral infection, methods for their use in treatment, and processes for their manufacture.

There is provided a recombinant adenovirus comprising:
a) a polynucleotide which encodes a polypeptide having the amino acid sequence according to SEQ ID NO: 1 and
b) a polynucleotide which encodes a polypeptide having the amino acid sequence according to SEQ ID NO: 3 and
c) a polynucleotide which encodes a polypeptide having the amino acid sequence according to SEQ ID NO: 5;

wherein the adenovirus comprises a nucleic acid sequence encoding a *Lyssavirus* antigen, wherein the nucleic acid sequence is operatively linked to one or more sequences which direct expression of said *Lyssavirus* antigen in a host cell;
wherein the nucleic acid sequence encoding a *Lyssavirus* antigen encodes an antigen derived from the rabies viral glycoprotein (G).

The recombinant adenoviruses and compositions may be for use as a medicament, in particular for the stimulation of an immune response against *Lyssaviral* diseases, such as a rabies infection.

Typically, the invention is for use to induce a protective immune response, *i.e.* to immunize or vaccinate the subject against a related pathogen. The invention may therefore be for use in the prophylaxis, treatment or amelioration of diseases due to infection by *Lyssaviral* diseases, such as infection by a rabies virus.

In some aspects, the compositions disclosed herein are immunogenic compositions that when administered to a subject, induce a humoral and/or cellular immune response, *i.e.,* an immune response which specifically recognizes a naturally occurring *Lyssaviral* polypeptide. For example, an immunogenic composition may induce a memory T and/or B cell population relative to an untreated subject following viral infection, particularly in those embodiments where the composition comprises a nucleic acid comprising a sequence which encodes a *Lyssaviral* antigen.

The invention may be provided for the purpose of both pre-exposure prophylaxis and post-exposure prophylaxis to diseases caused by *Lyssaviral* diseases. In some embodiments, the subject has previously been vaccinated with a rabies vaccine. The approaches of the present invention may, for example, be utilised for a subject at least one year after rabies vaccination, at least two years after rabies vaccination, at least at least five years after rabies vaccination or at least ten years after rabies vaccination. The *Lyssavirus* antigen is an antigenic sequence, *i.e.* a sequence from a *Lyssavirus* protein which comprises at least one B or T cell epitope. Suitably the *Lyssavirus* antigen comprises at least one T cell epitope. In an embodiment of the invention the adenovirus comprises a nucleic acid sequence encoding a *Lyssavirus* antigen. In a specific embodiment of the invention, the adenovirus comprises a nucleic acid encoding a polypeptide derived from SEQ ID NO: 37. In another specific embodiment of the invention, the adenovirus comprises a nucleic acid derived from SEQ ID NO: 38.

In another embodiment of the invention, the adenovirus may comprise a nucleic acid encoding a polypeptide derived from SEQ ID NO: 39, SEQ ID NO: 41, SEQ ID NO: 43, or SEQ ID NO: 45. In a further specific embodiment of the invention, the adenovirus may comprise a nucleic acid derived from SEQ ID NO: 40, SEQ ID NO: 42, SEQ ID NO: 44 or SEQ ID NO: 46.

The elicited immune response may be an antigen specific B cell response, which produces neutralizing antibodies. The elicited immune response may be an antigen specific T cell response, which may be a systemic and/or a local response. The antigen specific T cell response may comprise a CD4+ T cell response, such as a response involving CD4+ T cells expressing a plurality of cytokines, e.g. IFNgamma, tumor necrosis factor-alpha (TNFalpha) and/or IL2. Alternatively, or additionally, the antigen specific T cell response comprises a CD8+ T cell response, such as a response involving CD8+ T cells expressing a plurality of cytokines, e.g., IFNgamma, TNFalpha and/or IL2.

### DESCRIPTION OF THE DRAWINGS

FIG. 1: Diagram of the rabies glycoprotein indicating the main antigenic epitopes. FIG. 1 discloses SEQ ID NOS 94, 64 and 78, respectively, in order of appearance.

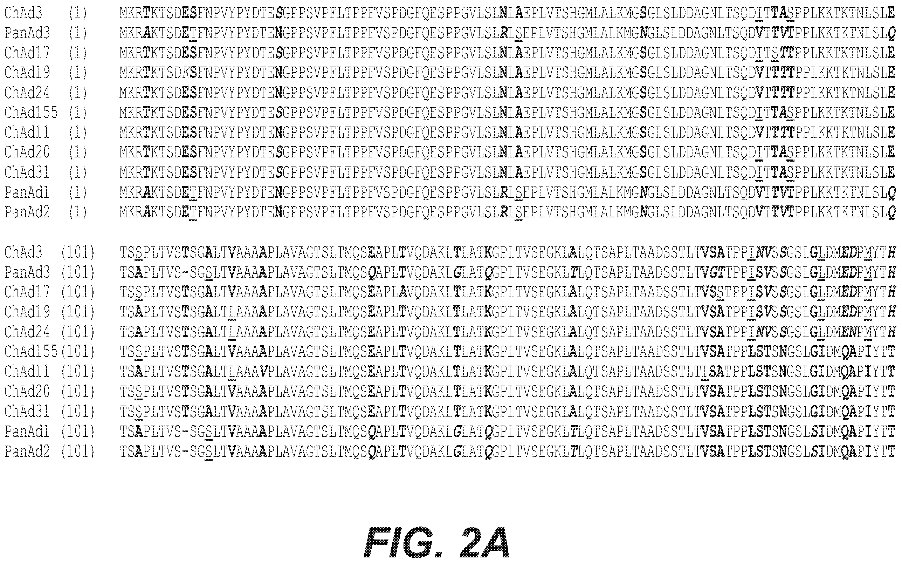

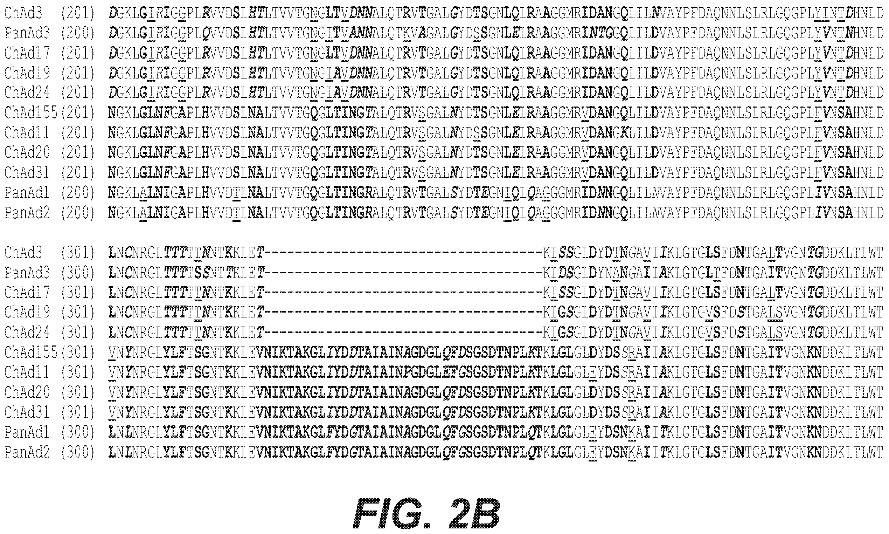

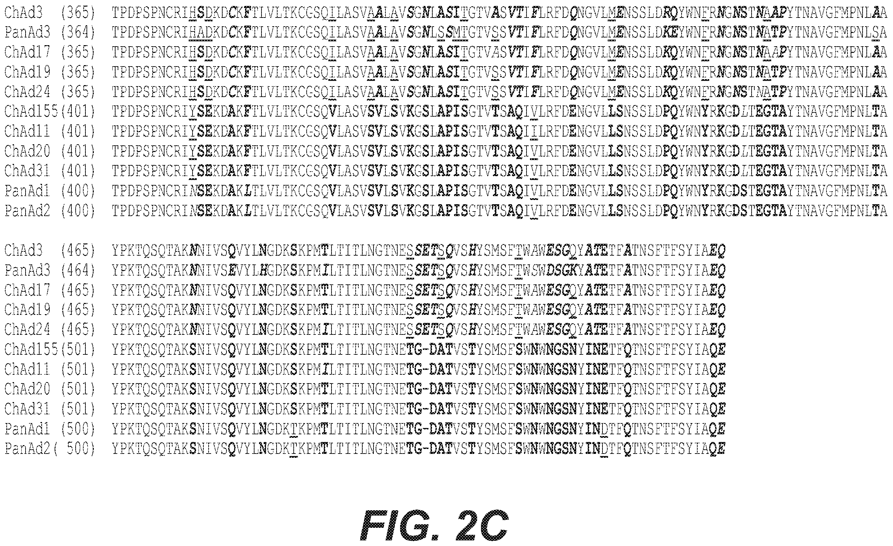

FIG. 2A-C: Alignment of fiber protein sequences from the indicated simian adenoviruses.
   ChAd3 (SEQ ID NO:27)
   PanAd3 (SEQ ID NO:28)
   ChAd17 (SEQ ID NO:29)
   ChAd19 (SEQ ID NO:30)
   ChAd24 (SEQ ID NO:31)
   ChAd155 (SEQ ID NO:1)
   ChAd11 (SEQ ID NO:32)
   ChAd20 (SEQ ID NO:33)
   ChAd31 (SEQ ID NO:34)
   PanAd1 (SEQ ID NO:35)
   PanAd2 (SEQ ID NO:36)
FIG. 3: Flow diagram for the production of specific ChAd155 BAC and plasmid vectors
FIG. 4: Species C BAC Shuttle #1365 schematic
FIG. 5: pArsChAd155 Ad5E4orf6-2 (#1490) schematic
FIG. 6: pChAd155/RSV schematic
FIG. 7: BAC ChAd155/RSV schematic
FIG. 8: E4 Ad5E4orf6/ TetO hCMV RG WPRE (#1509) schematic
FIG. 9: Productivity of ChAd3 and ChAd155 vectors expressing an HIV Gag transgene
FIG. 10: Expression levels of ChAd155 and PanAd3 vectors expressing an RSV transgene - western blot
FIG. 11: Immunogenicity of ChAd3 and ChAd155 vectors expressing an HIV Gag transgene - IFN-gamma ELISpot
FIG. 12: Immunogenicity of PanAd3 and ChAd155 vectors expressing an RSV transgene - IFN-gamma ELISpot
FIG. 13: Immunogenicity of ChAd155 vector expressing a rabies G protein transgene in mice - (A) antibody neutralization and (B) IFN-gamma ELISpot
FIG. 14: Stability of the immune response induced by ChAd155 vector expressing a rabies G protein transgene
FIG. 15: Potency of ChAd155 vector expressing a rabies G protein transgene compared to commercially available vaccines
FIG. 16: Seroconversion and protection rates of ChAd155 vector expressing a rabies G protein transgene in mice
FIG. 17: Seroconversion and protection rates of ChAd155 vector expressing a rabies G protein transgene in mice
FIG. 18: Neutralizing antibody response to ChAd155 vector expressing a rabies G protein transgene in non-human primates
FIG. 19: T cell response to ChAd155 vector expressing a rabies G protein transgene in non-human primates

### DESCRIPTION OF THE SEQUENCES

SEQ ID NO: 1 - Polypeptide sequence of ChAd155 fiber
SEQ ID NO: 2 - Polynucleotide sequence encoding ChAd155 fiber
SEQ ID NO: 3 - Polypeptide sequence of ChAd155 penton
SEQ ID NO: 4 - Polynucleotide sequence encoding ChAd155 penton
SEQ ID NO: 5 - Polypeptide sequence of ChAd155 hexon
SEQ ID NO: 6 - Polynucleotide sequence encoding ChAd155 hexon
SEQ ID NO: 7 - Polynucleotide sequence encoding ChAd155#1434
SEQ ID NO: 8 - Polynucleotide sequence encoding ChAd155#1390
SEQ ID NO: 9 - Polynucleotide sequence encoding ChAd155#1375
SEQ ID NO: 10 - Polynucleotide sequence encoding wild type ChAd155
SEQ ID NO: 11 - Polynucleotide sequence encoding ChAd155/RSV
SEQ ID NO: 12 - Polynucleotide sequence encoding the CASI promoter
SEQ ID NO: 13 - Ad5orf6 primer 1 polynucleotide sequence
SEQ ID NO: 14 - Ad5orf6 primer 2 polynucleotide sequence
SEQ ID NO: 15 - BAC/CHAd155 ΔE1_TetO hCMV RpsL-Kana primer 1 polynucleotide sequence
SEQ ID NO: 16 - BAC/CHAd155 ΔE1_TetO hCMV RpsL-Kana (#1375) primer 2 polynucleotide sequence
SEQ ID NO: 17 - 1021-FW E4 Del Step1 primer polynucleotide sequence
SEQ ID NO: 18 - 1022-RW E4 Del Step1 primer polynucleotide sequence
SEQ ID NO: 19 - 1025-FW E4 Del Step2 primer polynucleotide sequence
SEQ ID NO: 20 - 1026-RW E4 Del Step2 primer polynucleotide sequence
SEQ ID NO: 21 - 91-SubMonte FW primer polynucleotide sequence
SEQ ID NO: 22 - 90-BghPolyA RW primer polynucleotide sequence
SEQ ID NO: 23 - CMVfor primer polynucleotide sequence
SEQ ID NO: 24 - CMVrev primer polynucleotide sequence
SEQ ID NO: 25 - CMVFAM-TAMRA qPCR probe polynucleotide sequence
SEQ ID NO: 26 - Woodchuck Hepatitis Virus Posttranscriptional Regulatory Element (WPRE) polynucleotide sequence
SEQ ID NO: 27 - Amino acid sequence for the fiber protein of ChAd3
SEQ ID NO: 28 - Amino acid sequence for the fiber protein of PanAd3
SEQ ID NO: 29 - Amino acid sequence for the fiber protein of ChAd17
SEQ ID NO: 30 - Amino acid sequence for the fiber protein of ChAd19
SEQ ID NO: 31 - Amino acid sequence for the fiber protein of ChAd24
SEQ ID NO: 32 - Amino acid sequence for the fiber protein of ChAd11
SEQ ID NO: 33 - Amino acid sequence for the fiber protein of ChAd20
SEQ ID NO: 34 - Amino acid sequence for the fiber protein of ChAd31
SEQ ID NO: 35 - Amino acid sequence for the fiber protein of PanAd1
SEQ ID NO: 36 - Amino acid sequence for the fiber protein of PanAd2
SEQ ID NO: 37 - Amino acid sequence for the RG medoid antigen
SEQ ID NO: 38 - Nucleotide sequence for the RG medoid antigen
SEQ ID NO: 39 - Amino acid sequence for RG AA098
SEQ ID NO: 40 - Nucleic acid sequence for RG AA098
SEQ ID NO: 41 - Amino acid sequence for RG AA0093
SEQ ID NO: 42 - Nucleic acid sequence for RG AA0093
SEQ ID NO: 43 - Amino acid sequence for RG AA0094
SEQ ID NO: 44 - Nucleic acid sequence for RG AA0094
SEQ ID NO: 45 - Amino acid sequence for RG AA0095
SEQ ID NO: 46 - Nucleic acid sequence for RG AA0095
SEQ ID NO: 47 - Amino acid sequence for AdC68rab.gp - ERA strain
SEQ ID NO: 48 - Nucleotide sequence for AdC68rab.gp - ERA strain
SEQ ID NO: 49 - Poly-histidine
SEQ ID NOS: 50-63 - Rabies G protein Site IIb antigenic epitopes
SEQ ID NOS: 64-77 - Rabies G protein Site I antigenic epitopes
SEQ ID NOS: 78-91 - Rabies G protein Site III antigenic epitopes
SEQ ID NO: 92 - pvjTetOhCMV_WPRE_BghPolyA forward primer
SEQ ID NO: 93 - pvjTetOhCMV_WPRE_BghPolyA reverse primer

### DETAILED DESCRIPTION OF THE INVENTION

### Adenoviral vectors

Adenovirus has been widely used for gene transfer applications due to its proven safety, ability to achieve highly efficient gene transfer in a variety of target tissues and large transgene capacity. Adenoviral vectors may be derived from a range of mammalian hosts. Over 100 distinct serotypes of adenovirus have been isolated which infect various mammalian species. These adenoviral serotypes have been categorized into six subgenera (A-F; B is subdivided into B1 and B2) according to sequence homology and on their ability to agglutinate red blood cells.

Numerous adenoviruses have been isolated from nonhuman simians such as chimpanzees, bonobos, rhesus macaques and gorillas, and vectors derived from these adenoviruses induce strong immune responses to transgenes encoded by these vectors (Colloca et al. (2012) Sci. Transl. Med. 4:1-9; Roy et al. (2004) Virol.324: 361-372; Roy et al. (2010) J. of Gene Med. 13:17-25). Certain advantages of vectors based on nonhuman simian adenoviruses include the relative lack of cross-neutralizing antibodies to these adenoviruses in the target population, thus their use overcomes the pre-existing immunity to human adenoviruses. For example, cross-reaction of certain chimpanzee adenoviruses with pre-existing neutralizing antibody responses is only present in 2% of the target population compared with 35% in the case of certain candidate human adenovirus vectors.

### Adenoviral vector structure

Adenoviruses have a characteristic morphology with an icosahedral capsid comprising three major proteins, hexon (II), penton base (III) and a knobbed fiber (IV), along with a number of other minor proteins, VI, VIII, IX, IIIa and IVa2. The hexon accounts for the majority of the structural components of the capsid, which consists of 240 trimeric hexon capsomeres and 12 penton bases. The hexon has three conserved double barrels, while the top has three towers, each tower containing a loop from each subunit that forms most of the capsid. The base of the hexon is highly conserved between adenoviral serotypes, while the surface loops are variable. The penton is another adenoviral capsid protein that forms a pentameric base to which the fiber attaches. The trimeric fiber protein protrudes from the penton base at each of the 12 vertices of the capsid and is a knobbed rod-like structure. The primary role of the fiber protein is the tethering of the viral capsid to the cell surface via the interaction of the knob region with a cellular receptor, and variations in the flexible shaft as well as knob regions of fiber are characteristic of the different serotypes.

The adenoviral genome has been well characterized. The linear, double-stranded DNA is associated with the highly basic protein VII and a small peptide pX (also termed mu). Another protein, V, is packaged with this DNA-protein complex and provides a structural link to the capsid via protein VI. There is general conservation in the overall organization of the adenoviral genome with respect to specific open reading frames being similarly positioned, e.g. the location of the E1A, E1B, E2A, E2B, E3, E4, L1, L2, L3, L4 and L5 genes of each virus. Each extremity of the adenoviral genome comprises a sequence known as an inverted terminal repeat (ITR), which is necessary for viral replication. The 5' end of the adenoviral genome contains the 5' cis-elements necessary for packaging and replication; i.e., the 5' ITR sequences (which function as origins of replication) and the native 5' packaging enhancer domains (that contain sequences necessary for packaging linear adenoviral genomes and enhancer elements for the E1 promoter). The 3' end of the adenoviral genome includes the 3' cis-elements (including the ITRs) necessary for packaging and encapsidation. The virus also comprises a virus-encoded protease, which is necessary for processing some of the structural proteins required to produce infectious virions.

The structure of the adenoviral genome is described on the basis of the order in which the viral genes are expressed following host cell transduction. More specifically, the viral genes are referred to as early (E) or late (L) genes according to whether transcription occurs prior to or after onset of DNA replication. In the early phase of transduction, the E1A, E1B, E2A, E2B, E3 and E4 genes of adenovirus are expressed to prepare the host cell for viral replication. During the late phase of infection, expression of the late genes L1-L5, which encode the structural components of the virus particles, is activated.

### Adenovirus capsid proteins and their encoding polynucleotides

As outlined above, the adenoviral capsid comprises three major proteins, hexon, penton and fiber. The hexon accounts for the majority of the structural components of the capsid, which consists of 240 trimeric hexon capsomeres and 12 penton bases. The hexon has three conserved double barrels, while the top has three towers, each tower containing a loop from each subunit that forms most of the capsid. The base of hexon is highly conserved between adenoviral serotypes, while the surface loops are variable.

The penton is another adenoviral capsid protein that forms a pentameric base to which fiber attaches. The trimeric fiber protein protrudes from the penton base at each of the 12 vertices of the capsid and is a knobbed rod-like structure. A remarkable difference in the surface of adenovirus capsids compared to that of most other icosahedral viruses is the presence of the long, thin fiber protein. The primary role of the fiber protein is the tethering of the viral capsid to the cell surface via its interaction with a cellular receptor.

The fiber proteins of many adenovirus serotypes share a common architecture: an N-terminal tail, a central shaft made of repeating sequences, and a C-terminal globular knob domain (or "head"). The central shaft domain consists of a variable number of beta-repeats. The beta-repeats connect to form an elongated structure of three intertwined spiralling strands that is highly rigid and stable. The shaft connects the N-terminal tail with the globular knob structure, which is responsible for interaction with the target cellular receptor. The globular nature of the adenovirus knob domain presents large surfaces for binding the receptor laterally and apically. The effect of this architecture is to project the receptor-binding site far from the virus capsid, thus freeing the virus from steric constraints presented by the relatively flat capsid surface.

Although fibers of many adenovirus serotypes have the same overall architecture, they have variable amino acid sequences that influence their function as well as structure. For example, a number of exposed regions on the surface of the fiber knob present an easily adaptable receptor binding site. The globular shape of the fiber knob allows receptors to bind at the sides of the knob or on top of the fiber knob. These binding sites typically lie on surface-exposed loops connecting beta-strands that are poorly conserved among human adenoviruses. The exposed side chains on these loops give the knob a variety of surface features while preserving the tertiary and quaternary structure. For example, the electrostatic potential and charge distributions at the knob surfaces can vary due to the wide range of isoelectric points in the fiber knob sequences, varying from a pl of approximately 9 for adenovirus "Ad" 8, Ad 19, and Ad 37 to approximately 5 for subgroup B adenoviruses. As a structurally complex virus ligand, the fiber protein allows the presentation of a variety of binding surfaces (knob) in a number of orientations and distances (shaft) from the viral capsid.

One of the most obvious variations between some serotypes is fiber length. Studies have shown that the length of the fiber shaft strongly influences the interaction of the knob and the virus with its target receptors. Further, fiber proteins between serotypes can also vary in their ability to bend. Although beta-repeats in the shaft form a highly stable and regular structure, electron microscopy (EM) studies have shown distinct hinges in the fiber. Analysis of the protein sequence from several adenovirus serotype fibers pinpoints a disruption in the repeating sequences of the shaft at the third betarepeat from the N-terminal tail, which correlates strongly with one of the hinges in the shaft, as seen by EM. The hinges in the fiber allow the knob to adopt a variety of orientations relative to the virus capsid, which may circumvent steric hindrances to receptor engagement requiring the correct presentation of the receptor binding site on the knob. For example, the rigid fibers of subgroup D Ads thus require a flexible receptor or one prepositioned for virus attachment, as they are unable to bend themselves.

The identification of specific cell receptors for different Ad serotypes and the knowledge of how they contribute to tissue tropism have been achieved through the use of fiber pseudotyping technology. Although Ads of some subgroups use *Coxsackievirus* and adenovirus receptor ("CAR") as a primary receptor, it is becoming clear that many Ads use alternate primary receptors, leading to vastly different tropism *in vitro* and *in vivo.* The fibers of these serotypes show clear differences in their primary and tertiary structures, such as fiber shaft rigidity, the length of the fiber shaft, and the lack of a CAR binding site and/or the putative HSPG binding motif, together with the differences in net charge within the fiber knob. Pseudotyping Ad 5 particles with an alternate fiber shaft and knob therefore provides an opportunity to remove important cell binding domains and, in addition, may allow more efficient (and potentially more cell-selective) transgene delivery to defined cell types compared to that achieved with Ad 5. Neutralization of fiber-pseudotyped Ad particles may also be reduced if the fibers used are from Ads with lower seroprevalence in humans or experimental models, a situation that favours successful administration of the vector. Furthermore, full length fiber as well as isolated fiber knob regions, but not hexon or penton alone, are capable of inducing dendritic cell maturation and are associated with induction of a potent CD8+ T cell response. Taken together, adenoviral fiber plays an important role in at least receptor-binding and immunogenicity of adenoviral vectors.

"Low seroprevalence" may mean having a reduced pre-existing neutralizing antibody level as compared to human adenovirus 5 (Ad5). Similarly or alternatively, "low seroprevalence" may mean less than about 20% seroprevalence, less than about 15% seroprevalence, less than about 10% seroprevalence, less than about 5% seroprevalence, less than about 4% seroprevalence, less than about 3% seroprevalence, less than about 2% seroprevalence, less than about 1% seroprevalence or no detectable seroprevalence. Seroprevalence can be measured as the percentage of individuals having a clinically relevant neutralizing titer (defined as a 50% neutralisation titer >200) using methods as described in Aste-Amézaga et al., Hum. Gene Ther. (2004) 15(3):293-304.

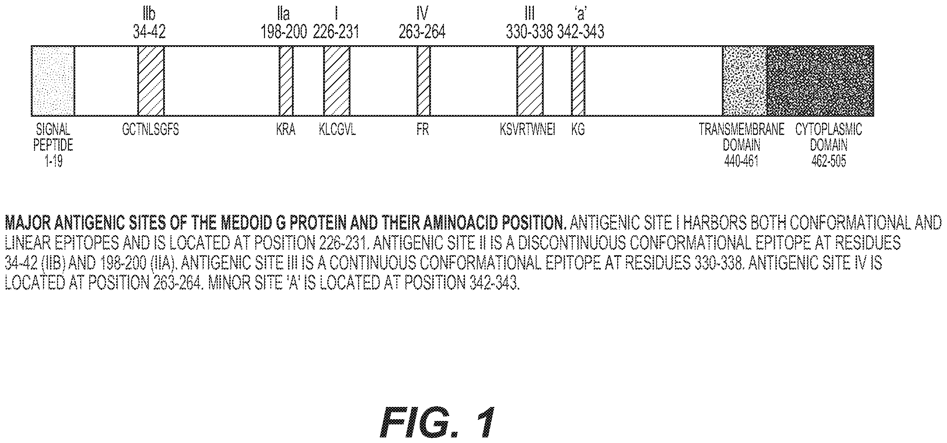

Illustrating the differences between the fiber proteins of Group C simian adenoviruses is the alignment provided in FIG. 1. A striking feature is that the fiber sequences of these adenoviruses can be broadly grouped into having a long fiber, such as ChAd155, or a short fiber, such as ChAd3. This length differential is due to a 36 amino acid deletion at approximately position 321 in the short fiber relative to the long fiber. In addition, there are a number of amino acid substitutions that differ between the short versus long fiber subgroup yet are consistent within each subgroup. While the exact function of these differences have not yet been elucidated, given the function and immunogenicity of fiber, they are likely to be significant. It has been shown that one of the determinants of viral tropism is the length of the fiber shaft. It has been demonstrated that an Ad5 vector with a shorter shaft has a lower efficiency of binding to CAR receptor and a lower infectivity. It has been speculated that this impairment is the result of an increased rigidity of the shorter fiber leading to a less efficient attachment to the cell receptor. These studies may explain the improved properties of ChAd155 carrying a longer and more flexible fiber in comparison with the previously described ChAd3 and PanAd3 carrying a fiber with a shorter shaft.

An "isolated" polynucleotide is one that is removed from its original environment. For example, a naturally-occurring polynucleotide is isolated if it is separated from some or all of the coexisting materials in the natural system. A polynucleotide is considered to be isolated if, for example, it is cloned into a vector that is not a part of its natural environment or if it is comprised within cDNA.

All three capsid proteins are expected to contribute to low seroprevalence.

### Transgenes

Adenoviral vectors may be used to deliver desired RNA or protein sequences, for example heterologous sequences, for *in vivo* expression. A vector may include any genetic element including naked DNA, a phage, transposon, cosmid, episome, plasmid, or virus. Such vectors contain DNA of ChAd155 as disclosed herein and an expression cassette. By "expression cassette" (or "minigene") is meant the combination of a selected heterologous gene ("transgene") and the other regulatory elements necessary to drive translation, transcription and/or expression of the gene product in a host cell.

Typically, "heterologous" means derived from a genotypically distinct entity from that of the rest of the entity to which it is being compared. A heterologous nucleic acid sequence refers to any nucleic acid sequence that is not isolated from, derived from, or based upon a naturally occurring nucleic acid sequence of the adenoviral vector. "Naturally occurring" means a sequence found in nature and not synthetically prepared or modified. A sequence is "derived" from a source when it is isolated from a source but modified (e.g., by deletion, substitution (mutation), insertion, or other modification), suitably so as not to disrupt the normal function of the source gene.

Typically, an adenoviral vector is designed such that the expression cassette is located in a nucleic acid molecule which contains other adenoviral sequences in the region native to a selected adenoviral gene. The expression cassette may be inserted into an existing gene region to disrupt the function of that region, if desired. Alternatively, the expression cassette may be inserted into the site of a partially or fully deleted adenoviral gene. For example, the expression cassette may be located in the site of a mutation, insertion or deletion which renders non-functional at least one gene of a genomic region selected from the group consisting of E1A, E1B, E2A, E2B, E3 and E4. The term "renders non-functional" means that a sufficient amount of the gene region is removed or otherwise disrupted, so that the gene region is no longer capable of producing functional products of gene expression. If desired, the entire gene region may be removed (and suitably replaced with the expression cassette). Suitably, E1 genes of adenovirus are deleted and replaced with an expression cassette consisting of a promoter of choice, a cDNA sequence of the gene of interest and a poly A signal, resulting in a replication defective recombinant virus.

The transgene encoded by the adenoviral vector is a sequence encoding a product which is useful in biology and medicine, such as one or more of a therapeutic or immunogenic protein or proteins, RNA or enzymes. Desirable RNA molecules include tRNA, dsRNA, ribosomal RNA, catalytic RNAs, RNA aptamers and antisense RNAs. An example of a useful RNA sequence is a sequence which extinguishes expression of a targeted nucleic acid sequence in the treated animal.

The transgene is a nucleic acid sequence, heterologous to the vector sequences flanking the transgene, which encodes a protein of interest. The nucleic acid coding sequence is operatively linked to regulatory components in a manner which permits transgene transcription, translation, and/or expression in a host cell.

The transgene may encode a polypeptide or protein used for disease treatment, amelioration or prophylaxis, for the induction of an immune response, and/or for prophylactic vaccine purposes. As used herein, induction of an immune response refers to the ability of a protein, also known as an "antigen" or "immunogen," to induce a T cell and/or a humoral immune response to the protein.

In an embodiment, the cross-protective breadth of a vaccine construct can be increased by comprising a medoid sequence of an antigen. By "medoid" is meant a *Lyssavirus* sequence with a minimal dissimilarity to other *Lyssavirus* sequences. In a particular embodiment, a vector of the invention comprises a medoid sequence of the G glycoprotein. In a particular embodiment, a non-human primate vector of the invention comprises a medoid sequence of the G glycoprotein. In a particular embodiment, a ChAd155 vector of the invention comprises a medoid sequence of the G glycoprotein. In a particular embodiment, the medoid sequence is derived from a natural viral strain with the highest average percent of amino acid identity among all G protein sequences annotated in the NCBI database. In a particular embodiment, the medoid sequence of the G glycoprotein is NCBI strain AGN94271.

Alternatively or in addition, a transgene sequence may include a reporter sequence, which upon expression produces a detectable signal. Such reporter sequences include, without limitation, DNA sequences encoding β-lactamase, β-galactosidase (LacZ), alkaline phosphatase, thymidine kinase, green fluorescent protein (GFP), chloramphenicol acetyltransferase (CAT), luciferase, membrane bound proteins including, for example, CD2, CD4, CD8, the influenza hemagglutinin protein, and others well known in the art, to which high affinity antibodies directed thereto exist or can be produced by conventional means, and fusion proteins comprising a membrane bound protein appropriately fused to an antigen tag domain from, among others, hemagglutinin or Myc. These coding sequences, when associated with regulatory elements which drive their expression, provide signals detectable by conventional means, including enzymatic, radiographic, colorimetric, fluorescence or other spectrographic assays, fluorescent activating cell sorting assays and immunological assays, including enzyme linked immunosorbent assay (ELISA), radioimmunoassay (RIA) and immunohistochemistry.

In addition to the transgene, the expression cassette also may include conventional control elements which are operably linked to the transgene in a manner that permits its transcription, translation and/or expression in a cell transfected with the adenoviral vector. As used herein, "operably linked" sequences include both expression control sequences that are contiguous with the gene of interest and expression control sequences that act in *trans* or at a distance to control the gene of interest.

Expression control sequences include appropriate transcription initiation, termination, promoter and enhancer sequences; efficient RNA processing signals such as splicing and polyadenylation (poly A) signals including rabbit beta-globin polyA; sequences that stabilize cytoplasmic mRNA; sequences that enhance translation efficiency (e.g., Kozak consensus sequence); sequences that enhance protein stability; and when desired, sequences that enhance secretion of the encoded product. Among other sequences, chimeric introns may be used.

A "promoter" is a nucleotide sequence that permits binding of RNA polymerase and directs the transcription of a gene. Typically, a promoter is located in the 5' non-coding region of a gene, proximal to the transcriptional start site of the gene. Sequence elements within promoters that function in the initiation of transcription are often characterized by consensus nucleotide sequences. Examples of promoters include, but are not limited to, promoters from bacteria, yeast, plants, viruses, and mammals (including humans). A great number of expression control sequences, including promoters which are internal, native, constitutive, inducible and/or tissue-specific, are known in the art and may be utilized.

Examples of constitutive promoters include, without limitation, the TBG promoter, the retroviral Rous sarcoma virus LTR promoter (optionally with the enhancer), the cytomegalovirus (CMV) promoter (optionally with the CMV enhancer, see, e.g., Boshart et al, Cell, 41:521-530 (1985)), the CASI promoter (WO2012/115980), the SV40 promoter, the dihydrofolate reductase promoter, the β-actin promoter, the phosphoglycerol kinase (PGK) promoter, and the EF1a promoter (Invitrogen).

Inducible promoters allow regulation of gene expression and can be regulated by exogenously supplied compounds, environmental factors such as temperature, or the presence of a specific physiological state, e.g., acute phase, a particular differentiation state of the cell, or in replicating cells only. Inducible promoters and inducible systems are available from a variety of commercial sources, including, without limitation, Invitrogen, Clontech and Ariad. Many other systems have been described and can be readily selected by one of skill in the art. For example, inducible promoters include the zinc-inducible sheep metallothionine (MT) promoter and the dexamethasone (Dex)-inducible mouse mammary tumor virus (MMTV) promoter. Other inducible systems include the T7 polymerase promoter system; the ecdysone insect promoter, the tetracycline-repressible system and the tetracycline-inducible system. Other systems include the FK506 dimer, VP16 or p65 using castradiol, diphenol murislerone, the RU486-inducible system and the rapamycin-inducible system. The effectiveness of some inducible promoters increases over time. In such cases one can enhance the effectiveness of such systems by inserting multiple repressors in tandem, e.g., TetR linked to a TetR by an IRES.

In another embodiment, the native promoter for the transgene may be used. The native promoter may be preferred when it is desired that expression of the transgene should mimic the native expression. The native promoter may be used when expression of the transgene must be regulated temporally or developmentally, or in a tissue-specific manner, or in response to specific transcriptional stimuli. In a further embodiment, other native expression control elements, such as enhancer elements, polyadenylation sites or Kozak consensus sequences may also be used to mimic the native expression.

The transgene may be operably linked to a tissue-specific promoter. For instance, if expression in skeletal muscle is desired, a promoter active in muscle should be used. These include the promoters from genes encoding skeletal β-actin, myosin light chain 2A, dystrophin, muscle creatine kinase, as well as synthetic muscle promoters with activities higher than naturally occurring promoters. Examples of promoters that are tissue-specific are known for liver; hepatitis B virus core; alpha-fetoprotein, bone osteocalcin; bone sialoprotein, lymphocytes, immunoglobulin heavy chain; T cell receptor chain), neuronal such as neuron-specific enolase (NSE) promoter, neurofilament light-chain gene, and the neuron-specific vgf gene, among others.

In some embodiments, the Woodchuck Hepatitis Virus Posttranscriptional Regulatory Element (WPRE) (Zuffrey et al. (1999) J. Virol.; 73(4):2886-9) may be operably linked to the transgene.

The transgene may be used for treatment, e.g., as a vaccine, for induction of an immune response, and/or for prophylactic vaccine purposes. As used herein, induction of an immune response refers to the ability of a protein to induce a T cell and/or a humoral immune response to the protein.

### Adenoviral vector construction

Adenoviral vectors are generated by modifying wild type adenovirus to express heterologous genes and/or delete or inactivate undesirable adenoviral sequences. Adenoviral vectors may also have altered replication competency. For example the vector may be replication defective or have limited replication such that it has a reduced ability to replicate in non-complementing cells, compared to the wild type virus. This may be brought about by mutating the virus e.g., by deleting a gene involved in replication, for example deleting the E1A, E1B, E3 or E4 gene.

The adenoviral vectors in accordance with the present invention may comprise a functional E1 deletion. Thus the adenoviral vectors according to the invention may be replication defective due to the absence of the ability to express adenoviral E1A and/or E1B. The recombinant adenoviruses may also bear functional deletions in other genes for example, deletions in E3 or E4 genes. The adenovirus delayed early gene E3 may be eliminated from the adenovirus sequence which forms part of the recombinant virus. The function of E3 is not necessary to the production of the recombinant adenovirus particle. Thus, it is unnecessary to replace the function of this gene product in order to package a recombinant adenovirus useful in the invention. In one particular embodiment the recombinant adenoviruses have functionally deleted E1 and E3 genes. The construction of such vectors is described in Roy et al., Human Gene Therapy 15:519-530, 2004.

Recombinant adenoviruses may also be constructed having a functional deletion of the E4 gene. In a particular embodiment, the recombinant adenoviruses have functionally deleted E1 and E4 genes as described in Colloca et al. (2012) Sci. Transl. Med. 4:1-9; Roy et al. (2004) Virol.324: 361-372. In some embodiments, it may be desirable to retain the E4 ORF6 function. In one embodiment, the E4 ORF6 region may be replaced by a heterologous E4 ORF6, such as from human adenovirus 5 (Ad5). Thus, in one particular embodiment, the adenoviral vector may be functionally deleted in E1 and have the E4 ORF6 region from Ad5. Adenovirus vectors according to the invention may also contain a functional deletion in the delayed early gene E2a. Deletions may also be made in any of the late genes L1 through to L5 of the adenovirus genome. Similarly, deletions in the intermediate genes IX and IVa may be useful.

Other deletions may be made in the other structural or non-structural adenovirus genes. The above deletions may be used individually, e.g. an adenovirus sequence for use in the present invention may contain deletions of E1 only. Alternatively, deletions of entire genes or portions thereof effective to destroy their biological activity may be used in any combination. For example in one exemplary vector, the adenovirus sequences may have deletions of the E1 genes and the E4 gene, or of the E1, E2a and E3 genes, or of the E1 and E3 genes (such as functional deletions in E1a and E1b, and a deletion of at least part of E3), or of the E1, E2a and E4 genes, with or without deletion of E3 and so on. Such deletions may be partial or full deletions of these genes and may be used in combination with other mutations, such as temperature sensitive mutations to achieve a desired result.

These vectors are generated using techniques known to those of skill in the art. Such techniques include conventional cDNA cloning techniques such as those described in texts, the use of overlapping oligonucleotide sequences of the adenovirus genomes, polymerase chain reaction, and any suitable method which provides the desired nucleotide sequence. Particularly suitable methods include standard homologous recombination methods such as those provided in Colloca et al. (2012) Sci. Transl. Med. 4:1-9; Roy et al. (2004) Virol.324: 361-372; Roy et al. (2010) J. of Gene Med. 13:17-25; and WO2010/085984 or recombineering methods as described in Warming et al. Nuc. Acids Res. (2005) 33:e36.

### Adenoviral vector production

The adenoviral vectors can be produced in any suitable cell line in which the virus is capable of replication. In particular, complementing cell lines which provide the factors missing from the viral vector that result in its impaired replication characteristics (such as E1) can be used. Without limitation, such a cell line may be HeLa (ATCC Accession No. CCL 2), A549 (ATCC Accession No. CCL 185), HEK 293, KB (CCL 17), Detroit (e.g., Detroit 510, CCL 72) and WI-38 (CCL 75) cells, among others. These cell lines are all available from the American Type Culture Collection, 10801 University Boulevard, Manassas, Virginia 20110-2209, USA. Other suitable parent cell lines may be obtained from other sources, such as PGK-E1 retinoblasts, e.g., PER.C6^{™} cells, as represented by the cells deposited under ECACC no. 96022940 at the European Collection of Animal Cell Cultures (ECACC) at the Centre for Applied Microbiology and Research (CAMR, UK) or Her 96 cells (Crucell).

In many circumstances, a cell line expressing the one or more missing genes which are essential to the replication and infectivity of the virus, such as human E1, can be used to transcomplement a chimp adenoviral vector. This is particularly advantageous because, due to the diversity between the chimp adenovirus sequences of the invention and the human adenovirus sequences found in currently available packaging cells, the use of the current human E1-containing cells prevents the generation of replication-competent adenoviruses during the replication and production process.

Alternatively, if desired, one may utilize the sequences provided herein to generate a packaging cell or cell line that expresses, at a minimum, the E1 gene from ChAd155 under the transcriptional control of a promoter for expression in a selected parent cell line. Inducible or constitutive promoters may be employed for this purpose. Examples of such promoters are described in detail elsewhere in this document. A parent cell is selected for the generation of a novel cell line expressing any desired ChAd155 gene. Without limitation, such a parent cell line may be HeLa [ATCC Accession No. CCL 2], A549 [ATCC Accession No. CCL 185], HEK 293, KB [CCL 17], Detroit [e.g., Detroit 510, CCL 72] and WI-38 [CCL 75] cells, among others. These cell lines are all available from the American Type Culture Collection, 10801 University Boulevard, Manassas, Virginia 20110-2209, USA.

Such E1-expressing cell lines are useful in the generation of recombinant adenovirus E1 deleted vectors. Additionally, or alternatively, cell lines that express one or more adenoviral gene products, e.g., E1A, E1B, E2A, E3 and/or E4, can be constructed using essentially the same procedures as used in the generation of recombinant viral vectors. Such cell lines can be utilised to transcomplement adenovirus vectors deleted in the essential genes that encode those products, or to provide helper functions necessary for packaging of a helper-dependent virus (e.g., adeno-associated virus). The preparation of a host cell involves techniques such as the assembly of selected DNA sequences.

In an embodiment, the essential adenoviral gene products are provided in *trans* by the adenoviral vector and/or helper virus. In such an instance, a suitable host cell can be selected from any biological organism, including prokaryotic (e.g., bacterial) cells, and eukaryotic cells, including insect cells, yeast cells and mammalian cells.

Host cells may be selected from among any mammalian species, including, without limitation, cells such as A549, WEHI, 3T3, 10'l'l/2, HEK 293 cells or Per.C6 (the latter two of which express functional adenoviral E1), Saos, C2C12, L cells, HT1080, HepG2 and primary fibroblast, hepatocyte and myoblast cells derived from mammals including human, monkey, mouse, rat, rabbit, and hamster.

A particularly suitable complementation cell line is the Procell92 cell line. The Procell92 cell line is based on HEK 293 cells which express adenoviral E1 genes, transfected with the Tet repressor under control of the human phosphoglycerate kinase-1 (PGK) promoter, and the G418-resistance gene (Vitelli et al. PLOS One (2013) 8(e55435):1-9). Procell92.S is adapted for growth in suspension conditions and is also useful for producing adenoviral vectors expressing toxic proteins (www.okairos.com/e/inners.php?m=00084, last accessed 13 April 2015).

### Adenoviral delivery methods and dosage

The adenoviral vectors may be as administered in immunogenic compositions. An immunogenic composition as described herein is a composition comprising one or more recombinant vectors capable of inducing an immune response, for example a humoral (e.g., antibody) and/or cell-mediated (e.g., a cytotoxic T cell) response, against a transgene product delivered by the vector following delivery to a mammal, suitably a human. A recombinant adenovirus may comprise (suitably in any of its gene deletions) a gene encoding a desired immunogen and may therefore be used in a vaccine. The recombinant adenoviruses can be used as prophylactic or therapeutic vaccines against any pathogen for which the antigen(s) crucial for induction of an immune response, is able to limit the spread of the pathogen and for which cDNA is available.

Such vaccine or other immunogenic compositions may be formulated in a suitable delivery vehicle. The levels of immunity of the selected gene can be monitored to determine the need, if any, for boosters. Following an assessment of antibody titers in the serum, optional booster immunizations may be desired.

Optionally, a vaccine or immunogenic composition of the invention may be formulated to contain other components, including, e.g., adjuvants, stabilizers, pH adjusters, preservatives and the like. Examples of suitable adjuvants are provided below under "Adjuvants." Such an adjuvant can be administered with a priming DNA vaccine encoding an antigen to enhance the antigen-specific immune response compared with the immune response generated upon priming with a DNA vaccine encoding the antigen only. Alternatively, such an adjuvant can be administered with a polypeptide antigen which is administered in an administration regimen involving the vectors of the invention.

The adenoviral vector may be prepared for administration by being suspended or dissolved in a pharmaceutically or physiologically acceptable carrier such as isotonic saline, isotonic salt, solution or other formulations that will be apparent to those skilled in the art. The appropriate carrier will be evident to those skilled in the art and will depend in large part upon the route of administration. The compositions described herein may be administered to a mammal in a sustained release formulation using a biodegradable biocompatible polymer, or by on-site delivery using micelles, gels and liposomes.

In some embodiments, the recombinant adenovirus of the invention is administered to a subject by intramuscular injection, intravenous injection, intraperitoneal injection, subcutaneous injection, epicutaneous administration, intradermal administration, transdermal administration, intravaginal administration nasal administration, rectal administration or oral administration.

If the therapeutic regimen involves co-administration of one or more adenoviral vectors and a further component, each formulated in different compositions, they are favorably administered co-locationally at or near the same site. For example, the components can be administered (e.g. via an administration route selected from intramuscular, transdermal, intradermal, sub-cutaneous) to the same side or extremity ("co-lateral" administration) or to opposite sides or extremities ("contra-lateral" administration).

Dosages of the viral vector will depend primarily on factors such as the condition being treated, the severity of the condition being treated and the age, weight and health of the patient, thus may vary among patients. For example, a therapeutically effective adult human dosage of the viral vector generally contains 1x10⁵ to 1×10¹⁵ viral particles, such as from 1×10⁸ to 1×10¹² (e.g., 1×10⁸, 5×10⁸, 1×10⁹, 5×10⁹, 1×10¹⁰, 2.5×10¹⁰, 5×10¹⁰, 1×10¹¹ 5×10¹¹ or 1×10¹² particles). Alternatively, a viral vector can be administered at a dose that is typically from 1×10⁵ to 1×10¹⁰ plaque forming units (PFU), such as 1×10⁵ PFU, 5×10⁵ PFU, 1×10⁶ PFU, 5×10⁶ PFU, 1×10⁷ PFU, 5×10⁷ PFU, 1×10⁸ PFU, 5×10⁸ PFU, 1×10⁹ PFU, 5×10⁹ PFU, or 1×10¹⁰ PFU. Dosages will vary depending upon the size of the subject and the route of administration. For example, a suitable human dosage (for about an 80 kg subject) for intramuscular injection is in the range of about 1×10⁵ to about 5×10¹² particles per ml, for a single site. Optionally, multiple sites of administration may be used. In another example, a suitable human or veterinary dosage may be in the range of about 1×10⁷ to about 1×10¹⁵ particles for an oral formulation.

The adenoviral vector can be quantified by Quantitative PCR Analysis (Q-PCR), for example with primers and probes designed based on the CMV promoter region, using as the standard curve serial dilutions of plasmid DNA containing the vector genome with the expression cassette, including the human CMV (hCMV) promoter. The copy number in the test sample is determined by the parallel line analysis method. Alternative methods for vector particle quantification include analytical HPLC or spectrophotometric methods based on A₂₆₀ nm.

An immunologically effective amount of a nucleic acid may suitably be between 1 ng and 100 mg. For example, a suitable amount can be from 1 µg to 100 mg. By "immunologically effective amount" is meant that the administration of that amount to a subject is effective for inducing a measurable immune response against *Lyssavirus* in the subject.

An appropriate amount of the particular nucleic acid (e.g., vector) can readily be determined by those of skill in the art.

Exemplary effective amounts of a nucleic acid component can be between 1 ng and 100 µg, such as between 1 ng and 1 µg (e.g., 100 ng-1 µg), or between1 µg and 100 µg, such as 10 ng, 50 ng, 100 ng, 150 ng, 200 ng, 250 ng, 500 ng, 750 ng, or 1 µg. Effective amounts of a nucleic acid can also include from 1 µg to 500 µg, such as between 1 µg and 200 µg, such as between 10 and 100 µg, for example 1 µg, 2 µg, 5 µg, 10 µg, 20 µg, 50 µg, 75 µg, 100 µg, 150 µg, or 200 µg. Alternatively, an exemplary effective amount of a nucleic acid can be between 100 µg and 1 mg, such as from 100 µg to 500 µg, for example, 100 µg, 150 µg, 200 µg, 250 µg, 300 µg, 400 µg, 500 µg, 600 µg, 700 µg, 800 µg, 900 µg or 1 mg.

Generally a human dose will be in a volume of between 0.1 ml and 2ml, such as 0.5 ml and 2 ml. Thus the composition described herein can be formulated in a volume of, for example 0.1, 0.25, 0.5, 1.0, 1.5 or 2.0 ml human dose per individual or combined immunogenic components.

One of skill in the art may adjust these doses, depending on the route of administration and the therapeutic or vaccine application for which the recombinant vector is employed. The levels of expression of the transgene, or for an adjuvant, the level of circulating antibody, can be monitored to determine the frequency of dosage administration.

If one or more priming and/or boosting steps are used, this step may include a single dose that is administered hourly, daily, weekly or monthly, or yearly. As an example, mammals may receive one or two doses containing between about 10 µg to about 50 µg of plasmid in carrier. The amount or site of delivery is desirably selected based upon the identity and condition of the mammal.

The therapeutic level of, or the level of immune response against, the protein encoded by the selected transgene can be monitored to determine the need, if any, for boosters. Following an assessment of CD8+ T cell response, or optionally, antibody titers, in the serum, optional booster immunizations may be desired. Optionally, the adenoviral vector may be delivered in a single administration or in various combination regimens, e.g., in combination with a regimen or course of treatment involving other active ingredients or in a prime-boost regimen.

*Recombinant adenoviruses or compositions comprising polypeptide sequences* Recombinant means that the polynucleotide is the product of at least one of cloning, restriction or ligation steps, or other procedures that result in a polynucleotide that is distinct from a polynucleotide found in nature. A recombinant adenovirus is an adenovirus comprising a recombinant polynucleotide. A recombinant vector is a vector comprising a recombinant polynucleotide. A "recombinant virus" includes progeny of the original recombinant virus. A "recombinant vector" includes replicates of the original recombinant vector. A "recombinant polynucleotide" includes replicates of the original recombinant polynucleotide.

A "functional derivative" of a polypeptide suitably refers to a modified version of a polypeptide, e.g. wherein one or more amino acids of the polypeptide may be deleted, inserted, modified and/or substituted. A derivative of an unmodified adenoviral capsid protein is considered functional if, for example:
(a) an adenovirus comprising the derivative capsid protein within its capsid retains substantially the same or a lower seroprevalence compared to an adenovirus comprising the unmodified capsid protein and/or
(b) an adenovirus comprising the derivative capsid protein within its capsid retains substantially the same or a higher host cell infectivity compared to an adenovirus comprising the unmodified capsid protein and/or
(c) an adenovirus comprising the derivative capsid protein within its capsid retains substantially the same or a higher immunogenicity compared to an adenovirus comprising the unmodified capsid protein and/or
(d) an adenovirus comprising the derivative capsid protein within its capsid retains substantially the same or a higher level of transgene productivity compared to an adenovirus comprising the unmodified capsid protein.

### ChAd155 Backbones

The present application describes isolated polynucleotide sequences of chimp adenovirus ChAd155, including that of wild type, unmodified ChAd155 (SEQ ID NO: 10) and modified backbone constructs of ChAd155. These modified backbone constructs include ChAd155#1434 (SEQ ID NO: 7), ChAd155#1390 (SEQ ID NO: 8) and ChAd155#1375 (SEQ ID NO: 9). ChAd155 backbones may be used in the construction of recombinant replication-competent or replication-incompetent adenoviruses for the delivery of transgenes.

The term "construct" refers to a nucleic acid that encodes polypeptide sequences described herein and may comprise DNA or non-naturally occurring nucleic acid monomers.

The term "replication-competent" adenovirus refers to an adenovirus which can replicate in a host cell in the absence of any recombinant helper proteins comprised in the cell. Suitably, a "replication-competent" adenovirus comprises the following intact or functional essential early genes: E1A, E1B, E2A, E2B, E3 and E4. Wild type adenoviruses isolated from a particular animal will be replication competent in that animal.

The term "replication-incompetent" or "replication-defective" adenovirus refers to an adenovirus which is incapable of replication because it has been engineered to comprise at least a functional deletion (or "loss-of-function" mutation), i.e. a deletion or mutation which impairs the function of a gene without removing it entirely, e.g. introduction of artificial stop codons, deletion or mutation of active sites or interaction domains, mutation or deletion of a regulatory sequence of a gene etc., or a complete removal of a gene encoding a gene product that is essential for viral replication, such as one or more of the adenoviral genes selected from E1A, E1B, E2A, E2B, E3 and E4 (such as E3 ORF1, E3 ORF2, E3 ORF3, E3 ORF4, E3 ORF5, E3 ORF6, E3 ORF7, E3 ORF8, E3 ORF9, E4 ORF7, E4 ORF6, E4 ORF4, E4 ORF3, E4 ORF2 and/or E4 ORF1). Particularly suitably E1, and optionally E3 and/or E4, are deleted. If deleted, the aforementioned deleted gene region will suitably not be considered in the alignment when determining % identity with respect to another sequence.

The sequences of the invention are useful as therapeutic agents and in construction of a variety of vector systems, recombinant adenovirus and host cells. Suitably the term "vector" refers to a nucleic acid that has been substantially altered (e.g., a gene or functional region that has been deleted and/or inactivated) relative to a wild type sequence and/or incorporates a heterologous sequence, i.e., nucleic acid obtained from a different source (also called an "insert"), and replicating and/or expressing the inserted polynucleotide sequence, when introduced into a cell (e.g., a host cell). For example, the insert may be all or part of the ChAd155 sequences described herein. In addition or alternatively, a ChAd155 vector may be a ChAd155 adenovirus comprising one or more deletions or inactivations of viral genes, such as E1 or other viral gene or functional region described herein. Such a ChAd155, which may or may not comprise a heterologous sequence, is often called a "backbone" and may be used as is or as a starting point for additional modifications to the vector.

Annotation of the ChAd155 wild type sequence (SEQ ID NO: 10) sequence is provided below.

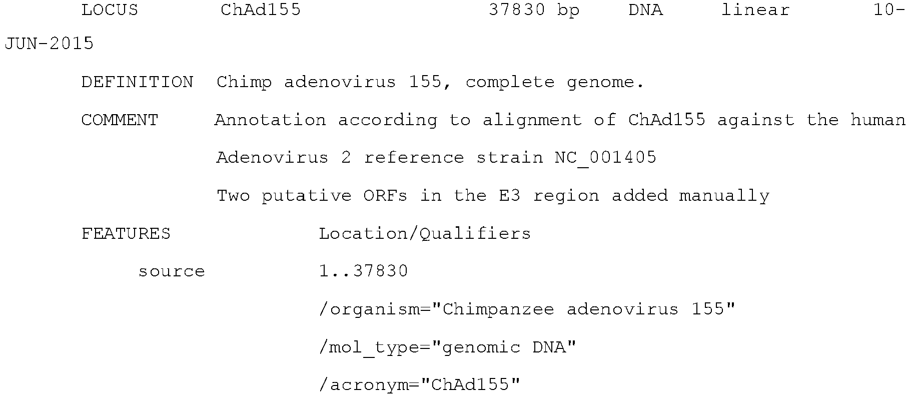

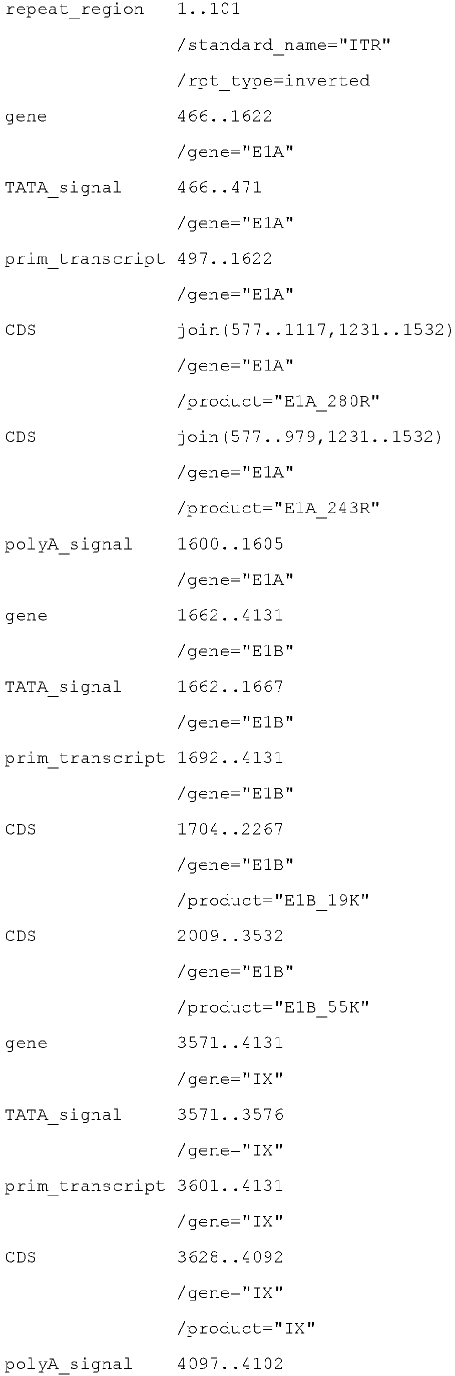

/note="E1B, IX"
gene complement(4117..27523)
/gene="E2B"
prim_transcript complement(4117..27494)
/gene="E2B"
gene complement(4117..5896)
/gene="IVa2"
prim_transcript complement(4117..5896)
/gene="IVa2"
CDS complement(join(4151..5487,5766..5778))
/gene="IVa2"
/product="E2B_IVa2"
polyA_signal complement(4150..4155)
/note="IVa2, E2B"
CDS complement(join(5257..8838,14209..14217))
/gene="E2B"
/product="E2B_polymerase"
gene 6078..34605
/gene="L5"
gene 6078..28612
/gene="L4"
gene 6078..22658
/gene="L3"
gene 6078..18164
/gene="L2"
gene 6078..14216
/gene="L1"
TATA_signal 6078..6083
/note="L"
prim_transcript 6109..34605
/gene="L5"
prim_transcript 6109..28612
/gene="L4"
prim_transcript 6109..22658
/gene="L3"
prim_transcript 6109..18164
/gene="L2"
prim_transcript 6109..14216
/gene="L1"

CDS                join(8038..8457,9722..9742)
                   /gene="L1"
                   /product="L1_13.6K"
CDS                complement(join(8637..10640,14209..14217))
                   /gene="E2B"
                   /product="E2B_pTP"
gene               10671..10832
                   /gene="VAI"
misc_RNA           10671..10832
                   /gene="VAI"
                   /product="VAI"
gene               10902..11072
                   /gene="VAII"
misc_RNA           10902..11072
                   /gene="VAII"
                   /product-"VAII"
CDS                11093..12352
                   /gene="L1"
                   /product="L1_52K"
CDS                12376..14157
                   /gene="L1"
                   /product="L1_pIIIa"
polyA_signal       14197..14202
                   /gene="L1"
CDS                14254..16035
                   /gene="L2"
                   /product="L2_penton"
CDS                16050..16646
                   /gene="L2"
                   /product="L2_pVII"
CDS                16719..17834
                   /gene="L2"
                   /product="L2_V"
CDS                17859..18104
                   /gene="L2"
                   /product="L2_pX"
polyA_signal       18143..18148
                   /gene="L2"
CDS                18196..18951

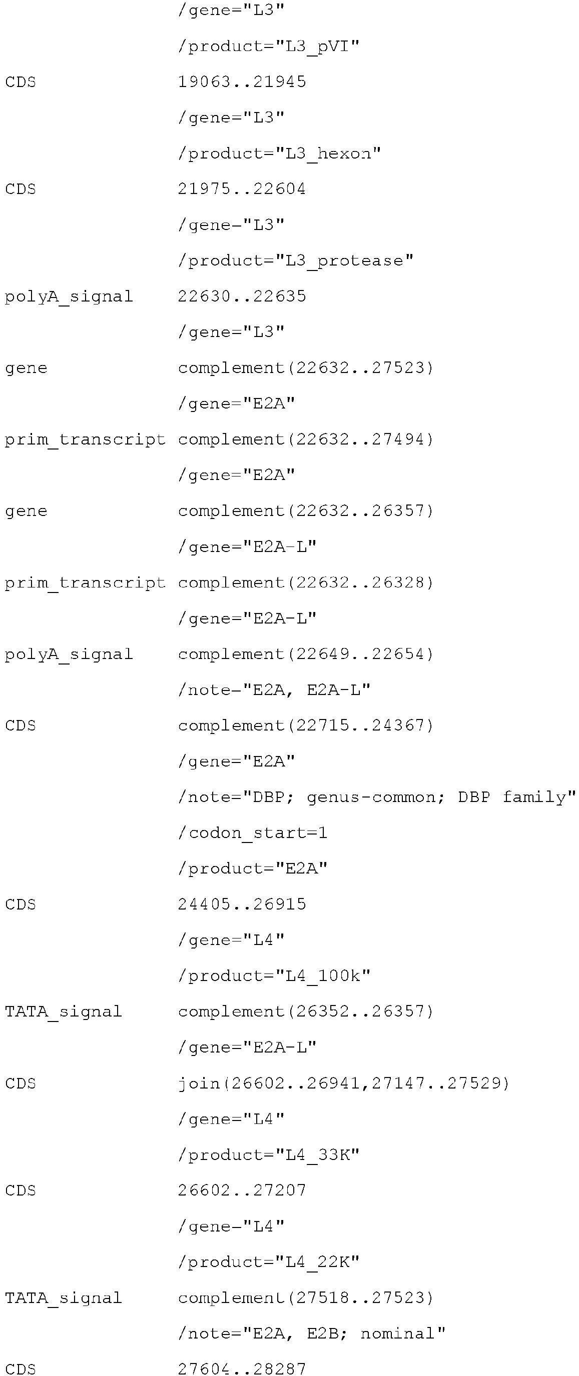

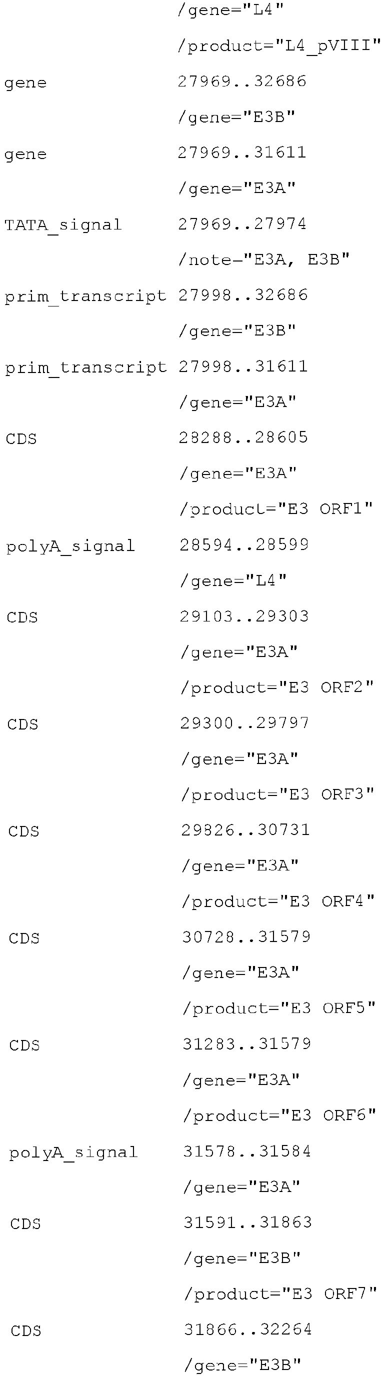

/product="E3 ORF8"
CDS 32257..32643
/gene="E3B"
/product="E3 ORF9"
polyA_signal 32659..32664
/gene="E3B"
gene complement(<32678..32838)
/gene="U"
CDS complement(<32678..32838)
/gene="U"
/note="exon encoding C terminus unidentified;
genus-common"
/product="protein U"
CDS 32849..34585
/gene="L5"
/product="L5_fiber"
polyA_signal 34581..34586
/gene="L5"
gene complement(34611..37520)
/gene="E4"
prim_transcript complement(34611..37490)
/gene="E4"
polyA_signal complement(34625..34630)
/gene="E4"
CDS complement(join(34794..35069,35781..35954))
/gene="E4"
/product="E4 ORF7"
CDS complement(35070..35954)
/gene="E4"
/product="E4 ORF6"
CDS complement(35875..36219)
/gene="E4"
/product="E4 ORF4"
CDS complement(36235..36582)
/gene="E4"
/product="E4 ORF3"
CDS complement(36579..36971)
/gene="E4"
/product="E4 ORF2"

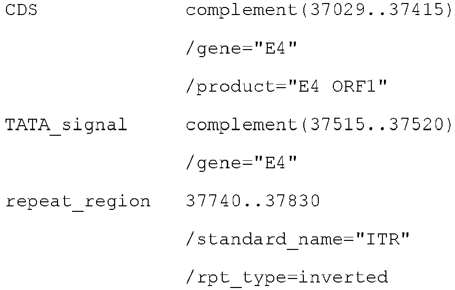

### Sequence identity

Identity with respect to a sequence is defined herein as the percentage of amino acid residues in the candidate sequence that are identical with the reference amino acid sequence after aligning the sequences and introducing gaps, if necessary, to achieve the maximum percent sequence identity, and not considering any conservative substitutions as part of the sequence identity.

Sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a pre-determined portion of one or both sequences). The programs provide a default opening penalty and a default gap penalty, and a scoring matrix such as PAM 250 (a standard scoring matrix can be used in conjunction with the computer program. For example, the percent identity can then be calculated as the total number of identical matches multiplied by 100 and then divided by the sum of the length of the longer sequence within the matched span and the number of gaps introduced into the shorter sequences in order to align the two sequences.

Where the present disclosure refers to a sequence by reference to a UniProt or Genbank accession code, the sequence referred to is the current version as of the filing date of the present application.

The skilled person will recognise that individual substitutions, deletions or additions to a protein which alters, adds or deletes a single amino acid or a small percentage of amino acids is an "immunogenic derivative" where the alteration(s) results in the substitution of an amino acid with a functionally similar amino acid or the substitution/deletion/addition of residues which do not substantially impact the immunogenic function.

Conservative substitution tables providing functionally similar amino acids are well known in the art. In general, such conservative substitutions will fall within one of the amino-acid groupings specified below, though in some circumstances other substitutions may be possible without substantially affecting the immunogenic properties of the antigen. The following eight groups each contain amino acids that are typically conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M).

Suitably such substitutions do not occur in the region of an epitope, and do not therefore have a significant impact on the immunogenic properties of the antigen.

Immunogenic derivatives may also include those wherein additional amino acids are inserted compared to the reference sequence. Suitably such insertions do not occur in the region of an epitope, and do not therefore have a significant impact on the immunogenic properties of the antigen. One example of insertions includes a short stretch of histidine residues (e.g. 2-6 residues) (SEQ ID NO: 49) to aid expression and/or purification of the antigen in question.

Immunogenic derivatives include those wherein amino acids have been deleted compared to the reference sequence. Suitably such deletions do not occur in the region of an epitope, and do not therefore have a significant impact on the immunogenic properties of the antigen.

The skilled person will recognise that a particular immunogenic derivative may comprise substitutions, deletions and additions (or any combination thereof).

### Lyssavirus antigens and vaccines

*Lyssavirus,* a genus in the *Rhabdoviridae* family, is an enveloped virus with a single stranded antisense RNA genome. The RNA encodes five structural proteins in the order of a nucleoprotein (N), a phosphoprotein (P), a matrix protein (M), a glycoprotein (G) and a viral RNA polymerase (L). The P protein is a structural component of the ribonucleoprotein and plays a role in the formation of viral particles and viral RNA synthesis. The G protein is thought to be important in viral pathogenicity and protective immunity; it is a major target of protective neutralizing antibodies. *Lyssavirus* is a neurotropic virus that spreads through the central nervous system causing severe inflammation of the brain and spinal cord.

The *Lyssavirus* genus comprises seven genotypes, the following six of which have been associated with cases of human rabies: rabies virus (RABV, genotype 1), Mokola virus (genotype 3), Duvenhage virus (genotype 4), European bat *Lyssavirus* (genotype 5), European bat *Lyssavirus* 2 (genotype 6), and Australian bat *Lyssavirus* (genotype 7) (Jackson (2016) Curr Infec Dis Rep 18:38). Once symptoms develop, rabies is nearly one hundred percent fatal.

Antigenic epitopes present on the rabies G protein have been identified in multiple strains of rabies viruses. They are classified as Site I, Site Ila, Site IIb, Site III, Site IV and Site a and are listed in Table 1. This Table discloses the 'Site II b' sequences as SEQ ID NOS 50-63, the 'Site I' sequences as SEQ ID NOS 64-77, and the 'Site III' sequences as SEQ ID NOS 78-91, respectively, in order of appearance.

**Table 1. Rabies G Protein Antigenic Epitopes**

| Virus | Phylo-group | Site II b (34-42) | Site II a (198-200) | Site I (226-231) | Site IV (263-264) | Site III (330-338) | Site 'a' (342-343) |
|---|---|---|---|---|---|---|---|
| RABV | I | GCTNLSEFS | KRA | KLCGVL | FH | KSVRTZNEI | KG |
| ABLV | I | GCTSLSGFS | KKA | KLCGIS | FH | KSVRTWDEI | KG |
| ARAV | I | GCTNLSGFT | KKA | KLCGVM | FH | KSVREWTEV | KG |
| BBLV | I | GCTTLTVFS | KKA | KLCGVS | FH | KSIRQWTEI | KG |
| DUVV | I | GCTTLTPFS | KKA | RLCGIS | FH | KSVREWKEI | KG |
| EBLV-1 | I | GCTTLTPFS | KKA | RLCGVP | FH | KSVREWKEV | KG |
| EBLV-2 | I | GCTTLTVFS | KKA | KLCGIS | FH | KSIREWTDV | KG |
| IRKV | I | GCTTLTAFN | KKA | KLCGMA | DR | KSIREWKEI | KG |
| KHUV | I | GCTTLSGFT | KRA | KLCGVS | FH | KSIREWSEI | KG |
| LBV | II | GCSDTATFS | KKS | TLCGKP | NR | LRVDSWNDI | KG |
| MOKV | II | GCNTESPFT | QKA | TLCGKP | DR | KRVDRWADI | KG |
| SHIV | II | GCSSSSTFS | KKS | TLCGKP | NR | KRVDRWEEI | KG |
| WCBV | III | YCTTEQSIT | KLV | SICGRQ | IK | IKVENWSEV | KG |
| IKOV | ? | GCNEGSKVS | ILL | IICGKS | VK | KSVDNWTDI | PI |

Antigenic epitopes present on the rabies G protein corresponding to SEQ ID NO: 37 are shown in FIG. 1. Antigenic Site I harbors both conformational and linear epitopes and is located at amino acid residues 226-231. Antigenic Site II is a discontinuous conformational epitope at residues 34-42 (Ilb) and 198-200 (IIa). Antigenic Site III is a continuous conformational epitope at residues 330-338. Antigenic Site IV is located at residues 263-264. Antigenic Site a is located at residues 342-343.

Rabies vaccines are currently used primarily for post-exposure prophylaxis, only a small percentage of rabies vaccine doses are used for pre-exposure prophylaxis. The intervention schedule is defined by the World Health Organization based on the seriousness and the type of the wound via which the virus gains entry and may include additional treatment with anti-rabies immunoglobulin. Pre-exposure prophylaxis typically involves two to three visits for two to three intramuscular doses with boosters timed according to the exposure risk. Post-exposure prophylaxis typically involves three to five visits for four to five intramuscular doses or four visits for four intradermal doses. In some less developed countries, immunization is still performed by propagating rabies virus in the brains of an infected animal, inactivating the virus and providing 14-21 daily injections given subcutaneously into the abdominal wall.

Several rabies vaccines are currently available for human use in both pre-exposure and post-exposure prophylaxis. IMOVAX (Sanofi Pasteur) is provided as freeze-dried rabies virus prepared from strain PM-1503-3M obtained from the Wistar Institute. It is harvested from infected human diploid cells then inactivated. Both pre- and post-exposure prophylaxis consists of three doses administered intramuscularly on days 0, 7 and 21 or 28. VERORAB (Sanofi Pasteur) is provided as freeze-dried rabies virus prepared from strain PM/WI 38 1503-3M obtained from the Wistar Institute. It is harvested from Vero cells then inactivated. Pre-exposure prophylaxis consists of three doses administered intramuscularly on days 0, 7 and 21 or 28. Post-exposure prophylaxis consists of five doses administered intramuscularly on days 0, 3, 7, 14 and 28. VAXIRAB/ LYSSAVAC (Zydus Cadila/ Novavax) is provided as freeze-dried rabies virus prepared from the Pitman Moore strain of the rabies virus. It is produced in duck embryo cells then inactivated. Pre-exposure prophylaxis consists of three doses administered intramuscularly on days 0, 7 and 21 or 28. Post-exposure prophylaxis consists of five doses administered intramuscularly on days 0, 3, 7, 14 and 28. Post-exposure prophylaxis can also be administered intradermally, injected at each of two sites on days 0, 3, 7 and 28. RABIPUR/ RABAVERT (GSK) is provided as a freeze-dried rabies virus prepared from the Flury LEP (low egg passage) strain. It is grown in primary cultures of chicken fibroblasts then inactivated. Pre-exposure prophylaxis consists of three doses administered intramuscularly on days 0, 7 and 21 or 28. Post-exposure prophylaxis consists of five doses administered intramuscularly on days 0, 3, 7, 14 and 28.

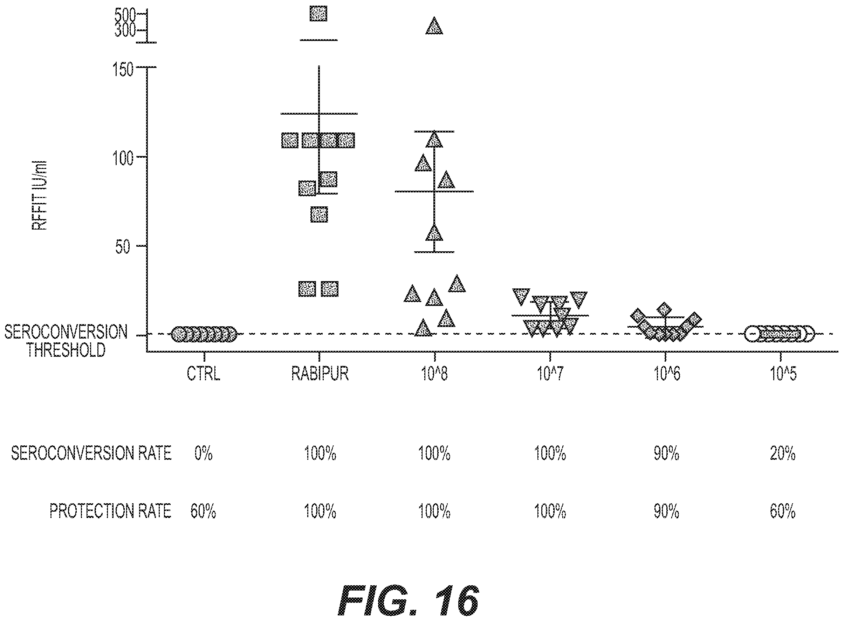

Supportive pre-clinical evidence for adeno-vectored rabies vaccines has been reported in the literature. The adenoviral recombinant viral vector SAdV24, also termed AdC68 or ChAd68, modified to be replication defective and to express the full length glycoprotein (G) of the Evelyn Rokitniki Abelseth (ERA) strain of rabies showed some degree of immunogenicity in cynomologous monkeys when given prior to a rabies challenge but did not provide reliable protection after a rabies exposure (Xiang et al. (2014) Virol. 450-451:243-249). A similar replication defective ChAd68 vector expressing the full length glycoprotein (G) of the Evelyn Rokitniki Abelseth (ERA) strain of rabies, given intramuscularly, induced a degree of protection against a rabies challenge (Zhou et al. (2006) Mol. Ther. 14:662-672; reproduced in part in FIG. 16).

### Adjuvants

An "adjuvant" as used herein refers to a composition that enhances the immune response to an immunogen. A composition according to the invention that comprises an adjuvant can be used as a vaccine, e.g. for human subjects. The adjuvant accelerates, prolongs and/or enhances the quality and/or strength of an immune response to an antigen/immunogen in comparison to the administration of the antigen alone, thus, reduces the quantity of antigen/immunogen necessary in any given vaccine, and/or the frequency of injection necessary in order to generate an adequate immune response to the antigen/immunogen of interest.

Examples of adjuvants that may be used in the context of the compositions of the invention include inorganic adjuvants (e.g. inorganic metal salts such as aluminum phosphate or aluminum hydroxide), gel-like precipitates of aluminum hydroxide (alum); AIPO₄; alhydrogel; bacterial products from the outer membrane of Gram-negative bacteria, in particular monophosphoryl lipid A (MPLA), lipopolysaccharides (LPS), muramyl dipeptides and derivatives thereof; Freund's incomplete adjuvant; liposomes, in particular neutral liposomes, liposomes containing the composition and optionally cytokines; AS01B, AS01E, AS02; non-ionic block copolymers; ISCOMATRIX adjuvant; unmethylated DNA comprising CpG dinucleotides (CpG motif), in particular CpG ODN with a phosphorothioate (PTO) backbone (CpG PTO ODN) or phosphodiester (PO) backbone (CpG PO ODN); synthetic lipopeptide derivatives, in particular PamsCys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II interferons, in particular interferon-gamma (IFN-gamma), TNF-alpha; 25-dihydroxyvitamin D3 (calcitriol); and synthetic oligopeptides, in particular MHCII-presented peptides. Non-ionic block polymers containing polyoxyethylene (POE) and polyoxypropylene (POP), such as POE-POP-POE block copolymers may be used as an adjuvant.

Additional examples of adjuvants include inorganic adjuvants (e.g. inorganic metal salts such as aluminium phosphate or aluminium hydroxide), organic adjuvants (e.g. saponins, such as QS21, or squalene), oil-based adjuvants (e.g. Freund's complete adjuvant and Freund's incomplete adjuvant), cytokines (e.g. IL-1β, IL-2, IL-7, IL-12, IL-18, GM-CFS, and INF-γ) particulate adjuvants (e.g. immuno-stimulatory complexes (ISCOMS), liposomes, biodegradable microspheres, virosomes, bacterial adjuvants (e.g. monophosphoryl lipid A, such as 3-de-O-acylated monophosphoryl lipid A (3D-MPL), or muramyl peptides), synthetic adjuvants (e.g. monophosphoryl lipid A (MPL), in particular 3-de-O-acylated monophosphoryl lipid A (3D-MPL and muramyl peptide analogues, or synthetic lipid A, and synthetic polynucleotides adjuvants, e.g., polyarginine or polylysine.

Saponins are also suitable adjuvants, for example, the saponin Quil A, derived from the bark of the South American tree Quillaja Saponaria Molina, and fractions thereof. Purified fractions of Quil A are also known as immunostimulants, such as squalene, QS21, QS17 and QS7, a non-haemolytic fraction of Quil-A. Combinations of QS21 and polysorbate or cyclodextrin are also suitable.

Another example of an adjuvant is an immunostimulatory oligonucleotide containing unmethylated cytosine-guanosine dinucleotide motifs present in DNA ("CpG"). CpG is known as an adjuvant when administered by both systemic and mucosal routes. When formulated into vaccines, it may be administered in free solution together with free antigen or covalently conjugated to an antigen or formulated with a carrier such as aluminium hydroxide.

Activation of specific receptors can stimulate an immune response. Such receptors are known to the skilled artisan and comprise, for example, cytokine receptors, in particular type I cytokine receptors, type II cytokine receptors, TNF receptors; and a vitamin D receptor acting as transcription factor; and the Toll-like receptors 1 (TLR1), TLR-2, TLR 3, TLR4, TLR5, TLR-6, TLR7, and TLR9. Agonists to such receptors have adjuvant activity, i.e., are immunostimulatory. Other suitable adjuvants include alkyl glucosaminide phosphates (AGPs) or pharmaceutically acceptable salts of AGPs. Some AGPs are TLR4 agonists, and some are TLR4 antagonists. An adjuvant of the composition of the present invention may be one or more Toll-like receptor agonists. In a more preferred embodiment, the adjuvant is a Toll-like receptor 4 agonist. In a particular preferred embodiment, the adjuvant is a Toll-like receptor 9 agonist.

Adjuvants such as those described above may be formulated together with carriers, such as liposomes, oil in water emulsions, and/or metallic salts (including aluminum salts such as aluminum hydroxide). For example, 3D-MPL may be formulated with aluminum hydroxide or oil in water emulsions; QS21 may be formulated with cholesterol containing liposomes, oil in water emulsions or alum; CpG may be formulated with alum or with other cationic carriers.

Combinations of adjuvants may be utilized in the present invention, in particular a combination of a monophosphoryl lipid A and a saponin derivative, more particularly the combination of QS21 and 3D-MPL or a composition where the QS21 is quenched in cholesterol-containing liposomes (DQ). Alternatively, a combination of CpG plus a saponin such as QS21 is an adjuvant suitable for use in the present invention, as is a potent adjuvant formulation involving QS21, 3D-MPL and tocopherol in an oil in water emulsion. Saponin adjuvants may be formulated in a liposome and combined with an immunostimulatory oligonucleotide. Thus, suitable adjuvant systems include, for example, a combination of monophosphoryl lipid A, preferably 3D-MPL, together with an aluminium salt. A further exemplary adjuvant comprises QS21 and/or MPL and/or CpG. QS21 may be quenched in cholesterol-containing liposomes.

The fusion of the invariant chain to an antigen which is comprised by an expression system used for vaccination increases the immune response against said antigen, if it is administered with an adenovirus. Accordingly, in one embodiment of the invention, the immunogenic transgene may be co-expressed with invariant chain in a recombinant ChAd155 viral vector.

### General

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. The term "plurality" refers to two or more. Additionally, numerical limitations given with respect to concentrations or levels of a substance, such as solution component concentrations or ratios thereof, and reaction conditions such as temperatures, pressures and cycle times are intended to be approximate. The term "about" used herein is intended to mean the amount ±10%.

The invention will be further described by reference to the following, examples and figures.

### EXAMPLES

### Example 1: Isolation of ChAd155

Wild type chimpanzee adenovirus type 155 (ChAd155) was isolated from a healthy young chimpanzee housed at the New Iberia Research Center facility (New Iberia Research Center, The University of Louisiana at Lafayette) using standard procedures as described in Colloca et al. (2012) Sci. Transl. Med. 4:1-9 and WO 2010086189, which describes adenoviral isolation and characterization techniques.

### Example 2: ChAd155-RG Vector Construction

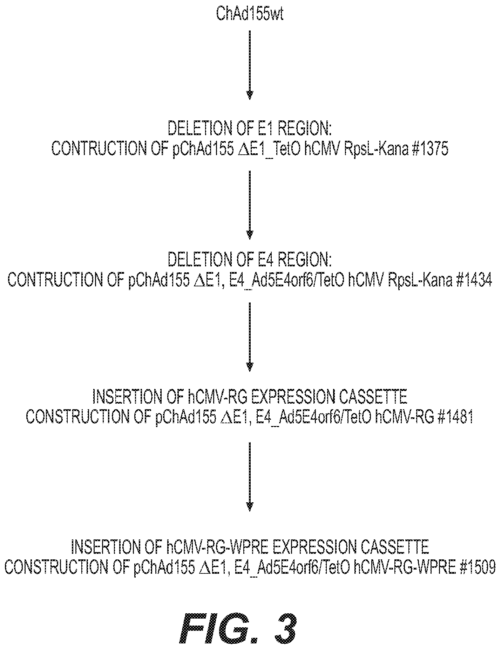

The ChAd155 viral genome was then cloned in a plasmid or in a BAC vector and subsequently modified as shown in FIG. 3:
a) deletion of the E1 region (from bp 449 to bp 3529) of the viral genome;
b) deletion of the E4 region (from bp 34731 to bp 37449) of the viral genome;
c) insertion of the E4orf6 derived from human Ad5; and
d) insertion of hCMV-RG-WPRE expression cassette.

### 2.1: Deletion of E1 region: Construction of BAC/ChAd155 ΔE1_TetO hCMV RpsL-Kana#1375

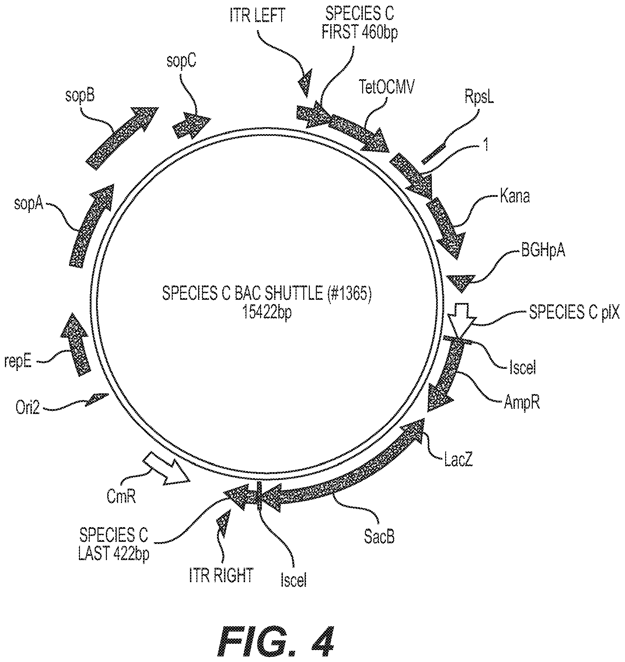

The ChAd155 viral genome was cloned into a BAC vector by homologous recombination in *E. coli* strain BJ5183 electroporation competent cells (Stratagene catalog no. 2000154) co-transformed with ChAd155 viral DNA and Subgroup C BAC Shuttle (#1365). As shown in the schematic of FIG. 4, the Subgroup C Shuttle is a BAC vector derived from pBeloBAC11 (GenBank U51113, NEB). It is dedicated to the cloning of chimp adeno viruses belonging to species C and therefore contains the pIX gene and DNA fragments derived from the right and left ends (including right and left ITRs) of species C ChAd viruses.

The Species C BAC Shuttle also contains a RpsL-Kana cassette inserted between the left end and the pIX gene. In addition, an Amp-LacZ-SacB selection cassette, flanked by IScel restriction sites, is present between the pIX gene and the right end of the viral genome. In particular, the BAC Shuttle comprised the following features: Left ITR: bp 27 to 139, hCMV(tetO) RpsL-Kana cassette: bp 493 to 3396, pIX gene: bp 3508 to 3972, IScel restriction sites: bp 3990 and 7481, Amp-LacZ-SacB selection cassette: bp 4000 to 7471, Right ITR: bp 7805 to 7917.

BJ5183 cells were co-transformed by electroporation with ChAd155 purified viral DNA and Subgroup C BAC Shuttle vector digested with IScel restriction enzyme and then gel purified. Homologous recombination occurring between pIX gene and right ITR sequences (present at the ends of Species C BAC Shuttle linearized DNA) and homologous sequences present in ChAd155 viral DNA lead to the insertion of ChAd155 viral genomic DNA in the BAC shuttle vector. At the same time, the viral E1 region was deleted and substituted by the RpsL-Kana cassette, generating BAC/ChAd155 ΔE1/ TetO hCMV RpsL-Kana#1375.

### 2.2: Plasmid construction by homologous recombination in E. coli BJ5183

### 2.2.1: Deletion of E4 region - Construction of pChAd155 ΔE1, E4_Ad5E4orf6/TetO hCMV RpsL-Kana (#1434)

To improve propagation of the vector, a deletion of the E4 region spanning from nucleotide 34731-37449 (ChAd155 wild type sequence) was introduced in the vector backbone by replacing the native E4 region with Ad5 E4orf6 coding sequence using a strategy involving several steps of cloning and homologous recombination in *E. coli.* The E4 coding region was completely deleted while the E4 native promoter and polyadenylation signal were conserved. To this end, a shuttle vector was constructed to allow the insertion of Ad5orf6 by replacing the ChAd155 native E4 region by homologous recombination in *E. coli* BJ5183 as detailed below.

### Construction of pARS SpeciesC Ad5E4orf6-1

A DNA fragment containing Ad5orf6 was obtained by PCR using Ad5 DNA as template, with the oligonucleotides 5'-ATACGGACTAGTGGAGAAGTACTCGCCTACATG-3' (SEQ ID NO: 13) and 5'-ATACGGAAGATCTAAGACTTCAGGAAATATGACTAC-3' (SEQ ID NO: 14). The PCR fragment was digested with Bglll and Spel and cloned into Species C RLD-EGFP shuttle digested with Bglll and Spel, generating the plasmid pARS Species C Ad5orf6-1. Details regarding the shuttle can be found in Colloca et al., Sci. Transl. Med. (2012) 4:115ra2.

### Construction of pARS SpeciesC Ad5E4orf6-2

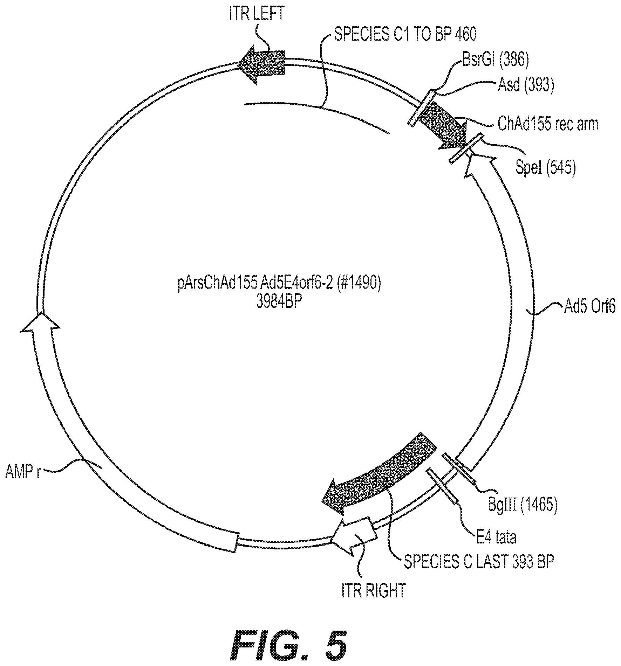

To delete the E4 region, a 177 bp DNA fragment spanning bp 34586 to bp 34730 of the ChAd155 wt sequence (SEQ ID NO: 10) was amplified by PCR using the plasmid BAC/ChAd155 ΔE1_TetO hCMV RpsL-Kana (#1375) as a template with the following oligonucleotides: 5'-ATTCAGTGTACAGGCGCGCCAAAGCATGACGCTGTTGATTTGATTC-3' (SEQ ID NO: 15) and 5'-ACTAGGACTAGTTATAAGCTAGAATGGGGCTTTGC-3' (SEQ ID NO: 16). The PCR fragment was digested with BsrGI and Spel and cloned into pARS SubGroupC Ad5orf6-1 digested with BsrGI and Spel, generating the plasmid pARS SpeciesC Ad5orf6-2 (#1490). A schematic diagram of this shuttle plasmid is provided in FIG. 5. In particular, the shuttle plasmid comprised the following features: Left ITR: bp 1 to 113, Species C first 460bp: bp 1 to 460, ChAd155 wt (bp 34587 to bp 34724 of SEQ ID NO:10) : bp 516 to 650, Ad5 orf6: bp 680 and 1561, Species C last 393 bp: bp 1567 to 1969, Right ITR: bp 1857 to 1969.

### Construction of pChAd155 ΔE1, E4_Ad5E4orf6/TetO hCMV RpsL-Kana (#1434)

The resulting plasmid pARS SubGroupC Ad5orf6-2 was then used to replace the E4 region within the ChAd155 backbone with Ad5orf6. To this end the plasmid BAC/ChAd155 ΔE1_TetO hCMV RpsL-Kana (#1375) was digested with Pacl/Pmel and co-transformed into BJ5183 cells with the digested plasmid pARS SubGroupC Ad5orf6-2 BsrGI/Ascl, to obtain the pChAd155 ΔE1, E4_Ad5E4orf6/TetO hCMV RpsL-Kana (#1434) pre-adeno plasmid.

### 2.2.2: Insertion of hCMV-RG Expression Cassette - Construction of pChAd155 ΔE1, E4_Ad5E4orf6/TetO hCMV-RG#1481

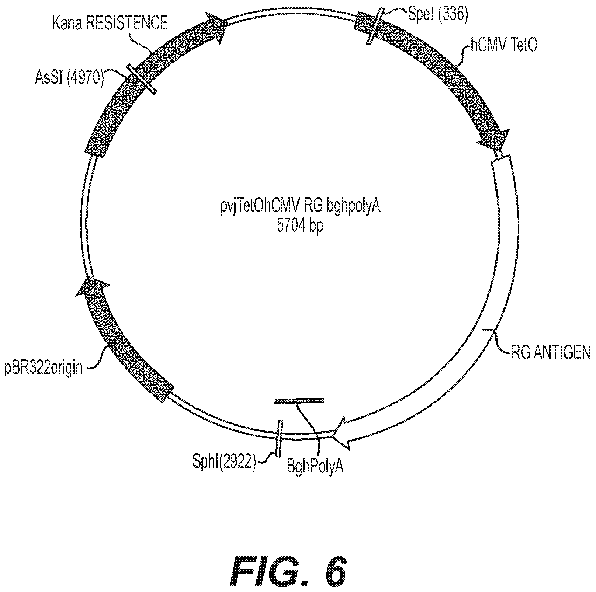

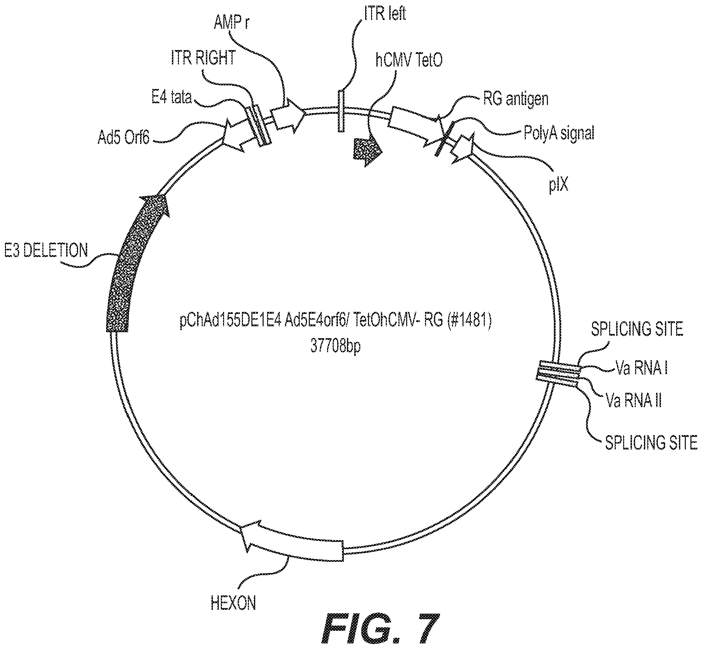

hCMV-RG cassette was cloned into a linearized pre-adeno acceptor vector via homologous recombination in *E. coli* by exploiting the homology existing between hCMV promoter and BGH polyA sequence. The plasmid pvjTetOhCMV_RG_bghpolyA, shown in FIG. 6, was cleaved with Spel, Sphl and AsiSI to excise the 2.58 Kb fragment containing the hCMV promoter with tetO, RG and BGHpolyA sequence. The resulting 2.58 Kb fragment was cloned by homologous recombination into the pChAd155 ΔE1, E4_Ad5E4orf6/TetO hCMV RpsL-Kana (#1434) acceptor vector carrying the RpsL-Kana selection cassette under the control of HCMV and BGHpA. The acceptor pre-adeno plasmid was linearized with the restriction endonuclease SnaBI. The resulting construct was the pChAd155 ΔE1, E4_Ad5E4orf6/TetO hCMV-RG vector (#1481) (FIG. 7).

### 2.2.3: Insertion of HCMV-RG-WPRE Expression Cassette - Construction of pChAd155 ΔE1, E4_Ad5E4orf6/TetO hCMV-RG-WPRE#1509

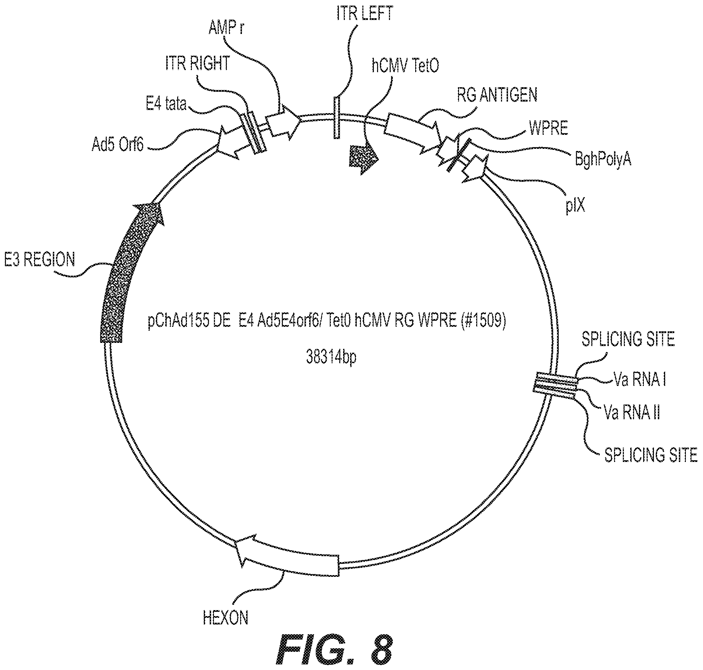

A WPRE sequence was cloned into a pre-adeno acceptor vector via homologous recombination in *E. coli* by exploiting the homology existing between bases 2840-2939 and 3180-3279 of pChAd155 ΔE1, E4_Ad5E4orf6/TetO hCMV-RG vector (#1481). A 1031 bp DNA fragment was amplified by PCR and contains WPRE, BGHpolyA and recombination arms corresponding to bases 2840-2939 and 3180-3279 of #1481 pAdeno vector. PCR was performed using the plasmid pvjTetOhCMV_WPRE_BghPolyA (#1478) as a template and with the following oligonucleotides FW 5'-ggaaggtcagcgtgaccagccagtccggcaaagtgatttcctcctgggagagctataaaagcggcggagagaccaggc tgtgatgagcggccgcgatctgtaatcaacctctggattaca -3' (SEQ ID NO: 92) and RW 5'-ATGGCTCCGGCGGTCTCTGCAACACAAATAAAGAGACCCTAAGACCCCCAACTTAT ATATTTTCATGACCACCCCAGGCCACGCCCACTCACCCACCTCACCATAGAGCCCA CCGCATCC-3' (SEQ ID NO: 93). The resulting 1.03 Kb fragment was cloned by homologous recombination into the pChAd155 ΔE1, E4_Ad5E4orf6/TetO hCMV-RG vector (#1481) acceptor vector carrying the RG transgene (SEQ ID NO: 38) under the control of hCMV promoter and BGHpA. The acceptor pre-adeno plasmid was digested with the restriction endonuclease AsiSl. The resulting construct was the pChAd155 ΔE1, E4_Ad5E4orf6/TetO hCMV-RG-WPRE vector (#1509), shown in FIG. 8.

### Example 3: ChAd155-RG Vector Production

The productivity of ChAd155 was evaluated in comparison to ChAd3 and PanAd3 in the Procell 92 cell line.

### 3.1: Production of vectors comprising an HIV Gag transgene

Vectors expressing the HIV Gag protein were prepared as described above (ChAd155/GAG) or previously as for ChAd3/GAG (Colloca et al, Sci. Transl. Med. (2012) 4:115ra2). ChAd3/GAG and ChAd155/GAG were rescued and amplified in Procell 92 until passage 3 (P3); P3 lysates were used to infect two T75 flasks of Procell 92 cells cultivated in monolayer with each vector. A multiplicity of infection (MOI) of 100 vp/cell was used for both infection experiments. The infected cells were harvested when the full cytopathic effect was evident (72 hours post-infection) and pooled; the viruses were released from the infected cells by three cycles of freeze/thaw (-70°/37°C) then the lysate was clarified by centrifugation. The clarified lysates were quantified by Quantitative PCR (QPCR) analysis with primers and probe complementary to the CMV promoter region. The oligonucleotide sequences are the following: CMVfor 5'-CATCTACGTATTAGTCATCGCTATTACCA-3' (SEQ ID NO: 23), CMVrev 5'-GACTTGGAAATCCCCGTGAGT-3' (SEQ ID NO: 24), CMVFAM-TAMRA probe 5'-ACATCAATGGGCGTGGATAGCGGTT-3' (SEQ ID NO: 25) (QPCRs were run on an ABI Prism 7900 Sequence detector-Applied Biosystem). The resulting volumetric titers (vp/ml) measured on clarified lysates and the cell specific productivity expressed in virus particles per cell (vp/cell) are provided in Table 2 below.

**Table 2. Vector productivity from P3 lysates**

| **Vector** | **vp/ml** | **Total vp (20 ml conc.)** | **vp/cell** |
|---|---|---|---|
| **ChAd3/GAG** | 9.82E+09 | 1.96E+11 | 6.61 E+03 |
| **ChAd155/GAG** | 1.11E+10 | 2.22E+11 | 7.46E+03 |

To confirm the higher productivity of the ChAd155 vector expressing HIV Gag transgene, a second experiment was performed by using purified viruses as inoculum. To this end, Procell 92 cells were seeded in a T25 Flask and infected with ChAd3/GAG and ChAd155/GAG when the confluence of the cells was about 80%, using an MOI=100 vp/cell. The infected cells were harvested when the full cytopathic effect was evident; the viruses were released from the infected cells by freeze/thaw and clarified by centrifugation. The clarified lysates were quantified by Quantitative PCR analysis by using the following primers and probe: CMVfor 5'-CATCTACGTATTAGTCATCGCTATTACCA-3' (SEQ ID NO: 23), CMV rev GACTTGGAAATCCCCGTGAGT (SEQ ID NO: 24), CMV FAM-TAMRA probe 5'-ACATCAATGGGCGTGGATAGCGGTT-3' (SEQ ID NO: 25) complementary to the CMV promoter region (samples were analysed on an ABI Prism 7900 Sequence detector-Applied Biosystems). The resulting volumetric titers (vp/ml) measured on clarified lysates and the cell specific productivity expressed in virus particles per cell (vp/cell) are provided in Table 3.

**Table 3. Vector productivity from purified viruses**

| **Vector** | **vp/ml** | **Total vp/T25 flask (5ml of lysate)** | **vp/cell** |
|---|---|---|---|
| **ChAd3/GAG** | 1.00E+10 | 5.00E+10 | 1.67E+04 |
| **ChAd155/GAG** | 1.21E+10 | 6.05E+10 | 2.02E+04 |

### 3.2: Production of vectors comprising an RSV transgene

A different set of experiments was performed to evaluate the productivity of RSV vaccine vectors in Procell 92.S cells cultivated in suspension. The experiment compared PanAd3/RSV (described in WO2012/089833) and ChAd155/RSV in parallel by infecting Procell 92.S at a cell density of 5x10⁵ cells/ml. The infected cells were harvested three days post infection; the virus was released from the infected cells by three cycles of freeze/thaw and the lysate was clarified by centrifugation. The clarified lysates were then quantified by Quantitative PCR analysis as reported above. The resulting volumetric titers (vp/ml) measured on clarified lysates and the cell specific productivity expressed in virus particles per cell (vp/cell) are provided in Table 4.

**Table 4. Vector productivity from purified viruses**

| **Virus** | **(Vp/ml)** | **Total vp** | **(vp/cell)** |
|---|---|---|---|
| **PanAd3/RSV** | 5.82E+09 | 2.91E+11 | 1.16E+4 |
| **ChAd155/RSV** | 3.16E+10 | 1.58E+12 | 6.31E+04 |

### Example 4: Transgene Expression Levels

### 4.1: Expression level of HIV Gag transgene

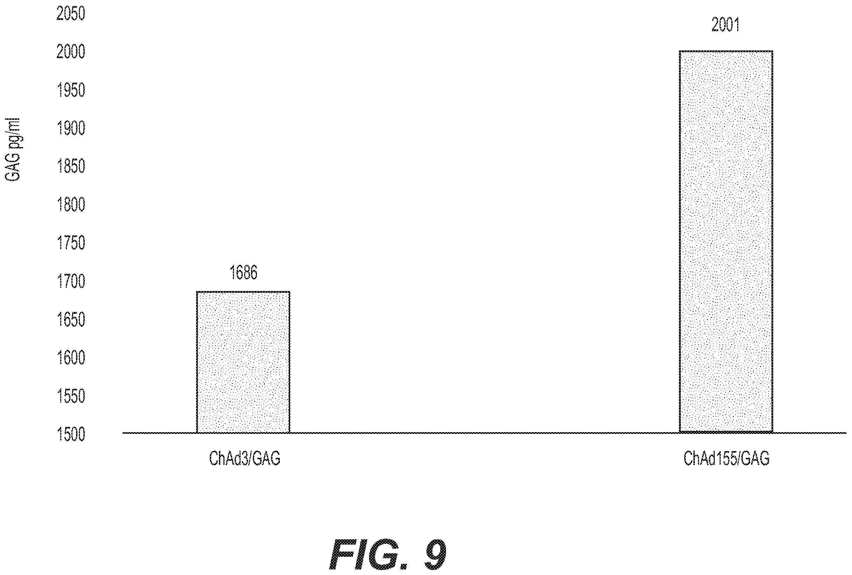

Expression levels were compared in parallel experiments by infecting HeLa cells with ChAd3 and ChAd155 vectors comprising an HIV Gag transgene. HeLa cells were seeded in 24 well plates and infected in duplicate with ChAd3/GAG and ChAd155/GAG purified viruses using an MOI=250 vp/cell. The supernatants of HeLa infected cells were harvested 48 hours post-infection, and the production of secreted HIV Gag protein was quantified by using a commercial ELISA Kit (HIV-1 p24 ELISA Kit, PerkinElmer Life Science). The quantification was performed according to the manufacturer's instruction by using an HIV-1 p24 antigen standard curve. The results, expressed in pg/ml of Gag protein, are illustrated in FIG. 9.

### 4.2: Expression level of RSV F transgene

Expression levels were compared in parallel experiments by infecting HeLa cells with the above-described PanAd3 and ChAd155 vectors comprising an RSV F transgene. To this end, HeLa cells were seeded in 6 well plates and infected in duplicate with PanAd3/RSV and ChAd155/RSV purified viruses using an MOI=500 vp/cell. The supernatants were harvested 48 hours post-infection, and the production of secreted RSV F protein was quantified by ELISA. Five different dilutions of the supernatants were transferred to microplate wells which were coated with a commercial mouse anti-RSV F monoclonal antibody. The captured antigen was revealed using a secondary anti-RSV F rabbit antiserum followed by biotin-conjugated anti-rabbit IgG, then by adding Streptavidin-AP conjugate (BD Pharmingen cat. 554065). The quantification was performed by using an RSV F protein (Sino Biological cat. 11049-V08B) standard curve. The results obtained, expressed as ug/ml of RSV F protein, are provided in Table 5.

**Table 5. Expression level of RSV F transgene**

| **Sample** | **µg/ml RSV F protein** |
|---|---|
| ChAd155/RSV | 5.9 |
| PanAd3/RSV | 4 |

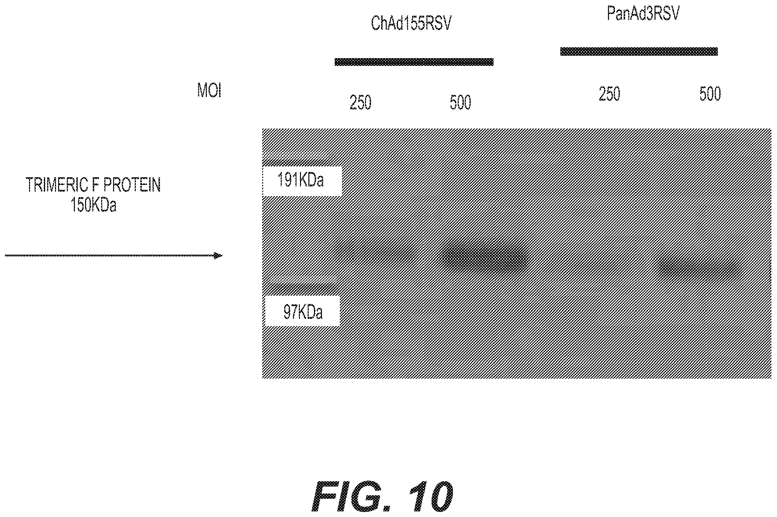

A western blot analysis was also performed to confirm the higher level of transgene expression provided by the ChAd155 RSV vector relative to the PanAd3 RSV vector. HeLa cells plated in 6 well plates were infected with PanAd3/RSV and ChAd155/RSV purified viruses using an MOI=250 and 500 vp/cell. The supernatants of HeLa infected cells were harvested and the production of secreted RSV F protein were analysed by non-reducing SDS gel electrophoresis followed by western blot analysis. Equivalent quantities of supernatants were loaded onto a non-reducing SDS gel; after electrophoresis separation, the proteins were transferred to a nitrocellulose membrane to be probed with an anti-RSV F mouse monoclonal antibody (clone RSV-F-3 catalog no: ABIN308230), available at antibodies-online.com (last accessed 13 April 2015). After the incubation with primary antibody, the membrane was washed and then incubated with anti-mouse HRP conjugate secondary antibody. Finally the assay was developed by electrochemiluminescence (ECL) using standard techniques (ECL detection reagents Pierce catalog no W3252282). The western blot results are shown in FIG. 10. A band of about 170 kD indicated by the arrow was revealed by monoclonal antibody mAb 13 raised against the F protein, which corresponds to the expected weight of trimeric F protein. It can be seen that the ChAd155 RSV vector produced a darker band than PanAd3RSV at MOls of both 250 and 500 vp/cell.

### Example 5: Evaluation of Immunological Potency by Mouse Immunization Experiments

### 5.1: Immunogenicity of vectors comprising the HIV Gag transgene

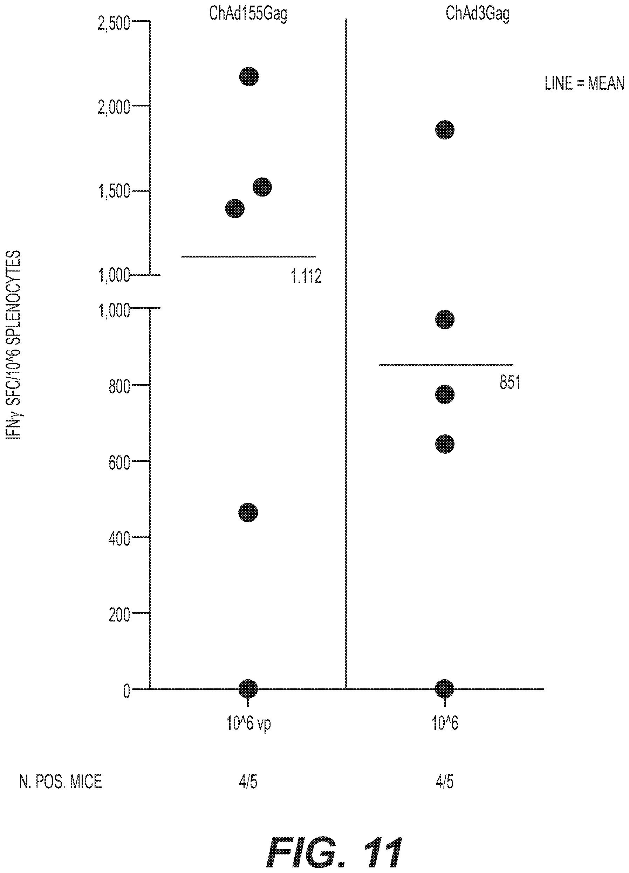

The immunogenicity of the ChAd155/GAG vector was evaluated in parallel with the ChAd3/GAG vector in BALB/c mice (five per group). The experiment was performed by injecting 10⁶ viral particles intramuscularly. T-cell response was measured three weeks after the immunization by ex *vivo* IFN-gamma enzyme-linked immunospot (ELISpot) using a GAG CD8+ T cell epitope mapped in BALB/c mice. The results are shown in FIG. 11, expressed as IFN-gamma Spot Forming Cells (SFC) per million splenocytes. Each dot represents the response in a single mouse, and the line corresponds to the mean for each dose group. Four out of five mice responded positively to the CD8 immunodominant peptide in response to both vectors.

### 5.2: Immunogenicity of vectors comprising the RSV transgene

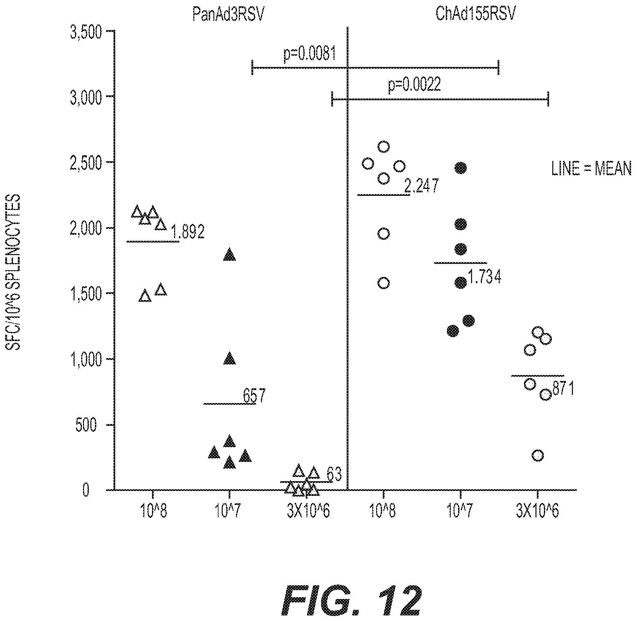

The immunological potency of the PanAd3/RSV and ChAd155/RSV vectors was evaluated in BALB/c mice. Both vectors were injected intramuscularly at doses of 10⁸, 10⁷ and 3×10⁶ vp. Three weeks after vaccination the splenocytes of immunized mice were isolated and analyzed by IFN-gamma-ELISpot using as antigens immunodominant peptide F and M epitopes mapped in BALB/c mice. The levels of the immune-responses were reduced in line with decreasing dosage (as expected) but immune responses were clearly higher in the groups of mice immunized with ChAd155/RSV vector compared to the equivalent groups of mice immunized with PanAd3/RSV vaccine (FIG. 12). Symbols show individual mouse data, expressed as IFN-gamma Spot Forming Cells (SFC)/million splenocytes, calculated as the sum of responses to the three immunodominant epitopes (F₅₁₋₆₆, F₈₅₋₉₃ and M2-1₂₈₂₋₂₉₀) and corrected for background. Horizontal lines represent the mean number of IFN-gamma SFC/million splenocytes for each dose group.

Taken together the results reported above demonstrated that ChAd155 is an improved adenoviral vector in comparison to ChAd3 and PanAd3 vectors. ChAd155 was shown to be more productive, therefore facilitating the manufacturing process, and shown to be able to express higher level of transgene both *in vitro* and *in vivo,* providing a stronger T-cell response against the antigens expressed in animal models.

### Example 6. ChAd155-RG Is Immunogenic and Protective Against a Rabies Challenge

### 6.1: Immunogenicity of the ChAd155-AG vector

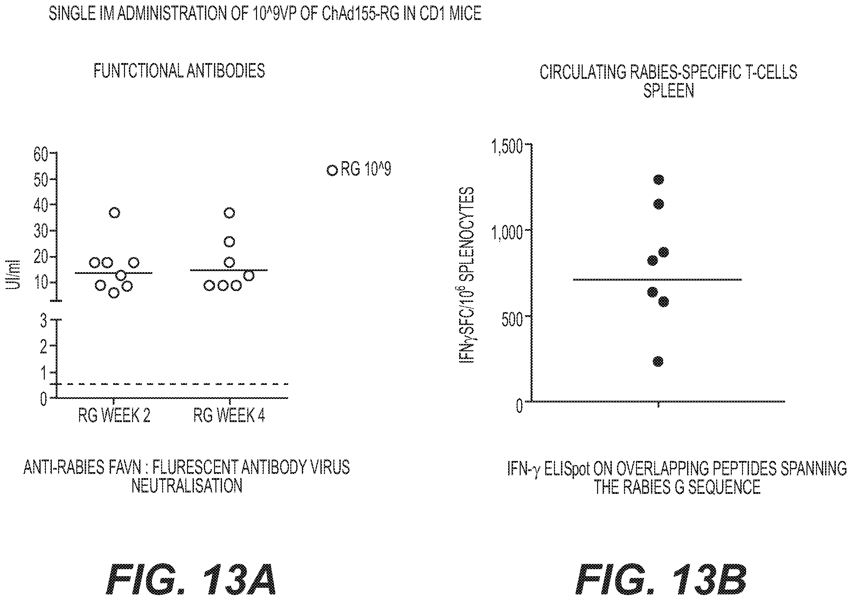

The immunological potency of the ChAd155-RG vector was evaluated in CD1 mice and the results shown in FIG. 13. The experiment was performed by injecting 10⁹ vp intramuscularly. Each dot represents the response of a single mouse. FIG. 13 demonstrates that a single administration of a replication defective adenoviral vector encoding the rabies viral G protein antigen induced a potent immune response. The vector induced protective levels of neutralizing antibodies (FIG. 13A) and induced circulating rabies specific T cells (FIG. 13B).

A fluorescent antibody virus neutralization assay (FAVN) was performed as described in Cliquet F. et al., J. Immunol. Methods (1998) 212:79-87. FIG. 13A demonstrates that functional neutralizing antibodies were detected in the serum within two weeks following a single administration of replication defective ChAd155-RG. Neutralizing antibodies were detected in amounts well above the protective threshold level of 0.5 IU/ml, as set forth in the World Health Organization guidelines (dotted line) by the second week post-administration showing no indication of a decline at week four. FIG. 13B demonstrates that rabies specific T cells were detected in the spleens of CD1 mice injected with 10⁻⁹ pfu/ml ChAd155-RG. An interferon-gamma ELISpot assay performed as described above on overlapping peptides spanning the rabies G protein sequence demonstrated the presence of rabies-specific T cells.

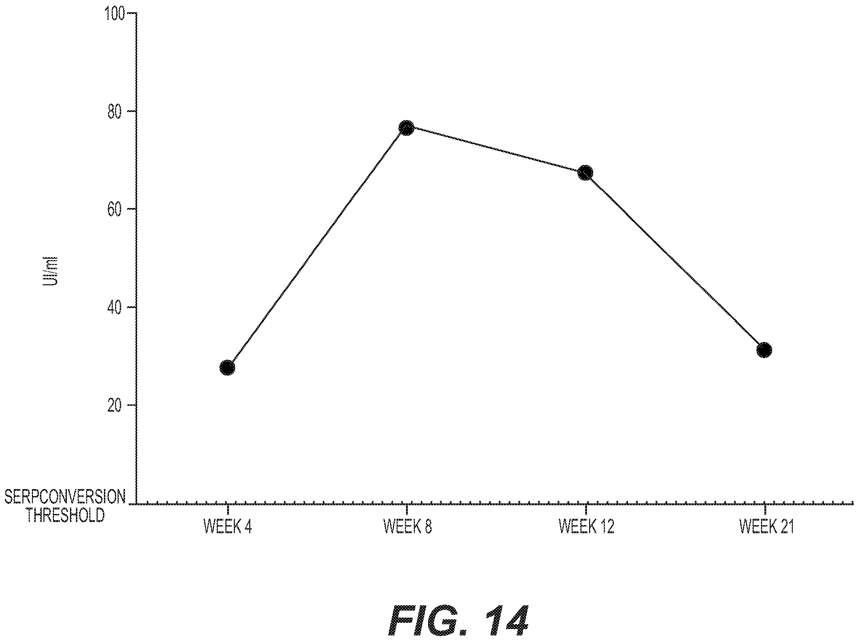

The antibody kinetics were followed up to 21 weeks after a single vaccine injection; the titers peaked at week 8 and then declined but remained well above the seroconversion threshold (dotted line), as shown in FIG. 14.

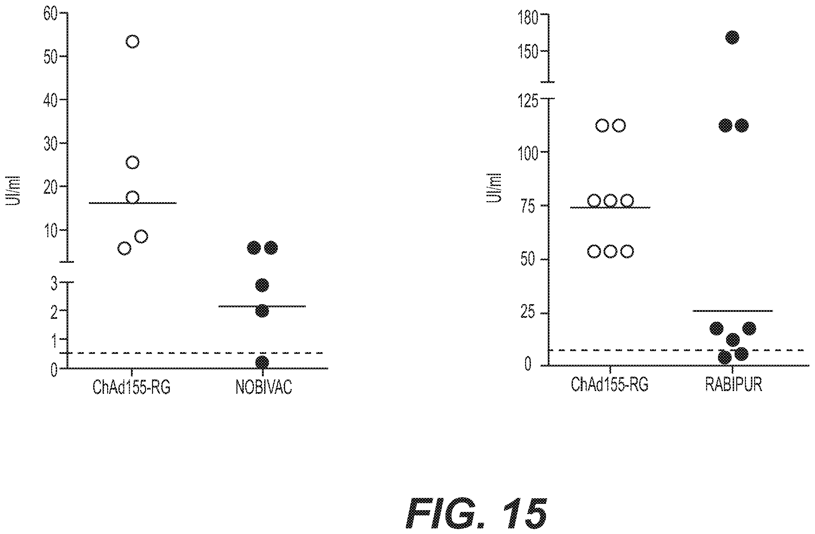

The immunological potency of ChAd155-RG was then compared to commercially available rabies vaccines in a single-dose regimen and the results are shown in FIG. 15. The left panel shows the results of immunizing Balb/c mice with either an estimated 1/500 of a human dose of ChAd155-RG (5x 10⁸ viral particles) or 1/10 of the canine dose of the veterinary rabies vaccine NOBIVAC. The right panel shows the results of immunizing CD1 mice with either 1/1000 of an estimated human dose of ChAd155-RG (10⁸ viral particles) or 1/10 of the human dose of RABIPUR. Virus neutralizing antibody titers were measured as described above, described as IU/ML, and the titers shown at two months after the single-dose vaccination. Despite the large excesses of the commercial vaccines, the immunity induced by the ChAd155-RG vector proved superior to both the commercially available veterinary and human rabies vaccines.

### 6.2: Ability of the ChAd155-AG vector to protect against a rabies challenge

FIG. 16 demonstrates that a single dose of ChAd155-RG protects against a rabies challenge. Outbred ICR mice, four to six weeks of age, were injected in the gastrocnemius muscle with ChAd155-RG, ChAd155 control vector or RABIPUR at the doses shown in Table 6. Each of groups 1-6 consisted of ten mice. The mice were given three doses of RABIPUR on days 0, 7 and 21 or a single dose of ChAd155 or ChAd155-RG at the doses shown in Table 6.

**Table 6. A Single Dose of ChAd155-RG Protects Against a Rabies Challenge**

| **Group** | **Vector** | **Dose** | **Seroconversion Rate** | **Survival Rate** |
|---|---|---|---|---|
| 1 | ChAd155 control | 10⁸ virus particles | 0% | 60% |
| 2 | RABIPUR at days 0, 7 and 21 | 1/10^{th} human dose x 3 | 100% | 100% |
| 3 | ChAd155-RG | 10⁸ virus particles | 100% | 100% |
| 4 | ChAd155-RG | 10⁷ virus particles | 100% | 100% |
| 5 | ChAd155-RG | 10⁶ virus particles | 90% | 90% |
| 6 | ChAd155-RG | 10⁵ virus particles | 20% | 60% |

The mice were then challenged with a human isolate of a bat rabies virus variant and followed for 90 days. The challenge virus was the street RABV variant Ps P4 isolated from a fatal human case associated with exposure to a rabid bat. The challenge dose, calculated in a previous experiment in naive unvaccinated animals, was 100% lethal. In this study, the same dose was 60% lethal. Serology was performed by a rapid fluorescent focus inhibition test for rabies (RFFIT), performed as described by Smith et al. (1973) Bull. World Health Organ. 48:535-541, to detect rabies-specific neutralizing antibodies. Also, direct immunofluorescence using LIGHT DIAGNOSTICS Rabies Polyclonal DFA Reagent (Millipore Cat#5199) was performed to detect viral antigen in the brain tissue.

FIG. 16 shows the level of rabies-specific neutralizing antibodies for each individual mouse. The mice in Group 1, given a negative control vector comprising no rabies antigen, did not seroconvert and 60% of the group survived. The mice in Groups 3-6 were given decreasing viral particle loads of ChAd155-RG. All of the mice seroconverted and survived when given 10⁸ or 10⁷ virus particles. Mice injected with 10⁶ virus particles had a 90% seroconversion rate and 90% survived. Mice injected with 10⁵ virus particles had a 20% seroconversion rate and 60% survived. This demonstrates that a single intramuscular vaccination of ChAd155-RG elicited neutralizing antibody titers above the threshold of 0.5 IU/ml over a wide dose range and conferred protection against a lethal rabies challenge. FIG. 16 and Table 6 therefore demonstrate that a single administration of recombinant ChAd155-RG can be at least as effective in protecting against rabies as a conventional, currently used, inactivated viral vaccine.

### Example 7. ChAd155-RG is More Potent than AdC68rab.gp in Protecting Against a Rabies Challenge

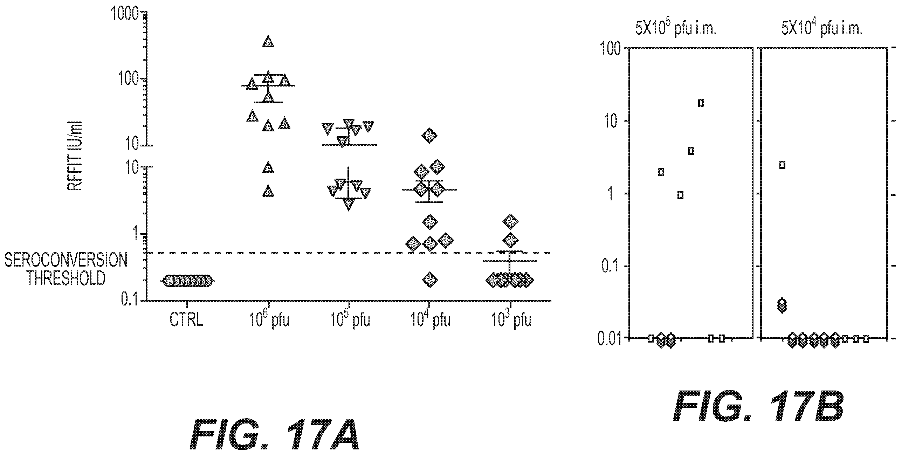

The potency of the ChAd155-RG vector to protect against a rabies virus challenge was compared to the potency, as reported in the literature, for the AdC68 rab.gp vector. Balb/c mice were immunized intramuscularly with a single dose of ChAd155-RG, as shown in Table 7. The pre-challenge viral neutralizing antibody levels were dosedependent and are shown in FIG. 17A. These mice were then challenged with a human isolate of a bat rabies virus variant, as described in Example 6. As shown in Table 7, 60% of the mice given control ChAd155 vector survived. Balb/c mice immunized with 10⁸ or 10⁷ vp ChAd155-RG had a 100% survival rate, mice immunized with 10⁶ vp ChAd155-RG had a 90% survival rate and mice immunized with 10⁶ vp ChAd155-RG had a 60% survival rate, and the neutralizing antibody titers fell to nearly the seroconversion threshold.

These results were then correlated with the results published by Zhou (2006) Mol. Ther. 14:662 at 670 (FIG. 17B). Zhou et al. reported immunizing ICR mice intramuscularly with the adenoviral recombinant viral vector AdC68rab.gp, then challenging intranasally with CVS-N2C rabies virus. Control animals were not vaccinated and had a 100% fatality rate. Forty five percent of the mice immunized with 5x10⁵ pfu AdC68rab.gp seroconverted and showed a 77% survival rate (17B left panel) while mice immunized with 5× 10⁴ pfu ChAd155-RG (17B right panel) showed 90% seroconversion and had a 60% survival rate.

Similar serological and protective efficacy data were obtained in the inventor' present study and the study reported by Zhou et al., when using a fifty-fold smaller dose (AdC68rab.gp at 5x10⁵ pfu compared to ChAd155-RG at 10⁴ pfu). ChAd155-RG is therefore about fifty times more potent than AdC68rab.gp.

**Table 7. Potency of ChAd155-RG and AdC68rab.gp**

| **Group** | **Vector** | **Dose** | **Seroconversion Rate** | **Survival Rate** |
|---|---|---|---|---|
| 1 | ChAd155 control | 10⁸ virus particles | 0% | 60% |
| 2 | ChAd155-RG | 10⁸ virus particles | 100% | 100% |
| 3 | ChAd155-RG | 10⁷ virus particles | 100% | 100% |
| 4 | ChAd155-RG | 10⁶ virus particles | 90% | 90% |
| 5 | ChAd155-RG | 10⁵ virus particles | 20% | 60% |
| 6 | AdC68rab.gp | 5×10⁷ virus particles | 44% | 77% |
| 7 | AdC68rab.gp | 5×10⁶ virus particles | 10% | 60% |

### Example 8. ChAd155-RG Provides Long-term Immunogenicity to Non-human Primates

To evaluate the kinetics, breadth and longevity of the immunogenicity of ChAd155-RG in non-human primates, three groups of five cynomologous monkeys (*Macaca fascicularis*) were treated as follows. Group 1 was immunized with ChAd155-RG 5x10¹⁰ viral particles IM followed by a booster dose of ChAd155-RG 5x10¹⁰ viral particles IM at week 48. Group 2 was immunized with ChAd155-RG 5x10¹⁰ viral particles IM, followed by a booster dose of RABIPUR vaccination at week 24 and a booster dose of ChAd155-RG 5x10¹⁰ viral particles IM at week 48. Group 3 received half of a human dose of RABIPUR administered intramuscularly and a booster dose of the same on days 7 and 21. Serum samples were collected at intervals and whole blood was collected for peripheral blood mononuclear cell (PBMC) analysis.

The immunogenicity, up to 48 weeks, induced by a single dose of ChAd155-RG was compared to a full course of RABIPUR. Boosts with either RABIPUR at week 24 or ChAd155 at week 48 were introduced to evaluate the compatibility of the two vaccines and the ability to boost medium to long term immune responses.

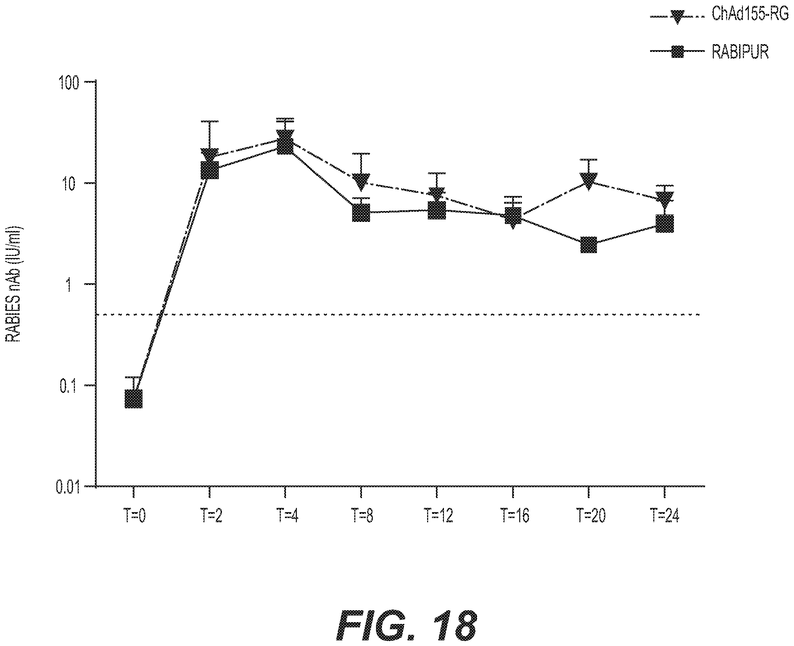

The neutralizing antibody titers induced by a single immunization with ChAd155-RG were compared to those induced with a full three dose course of RABIPUR. FIG. 18 shows the comparison of the neutralizing antibody responses, as measured by FAVN assay, of the monkeys immunized with recombinant ChAd155-RG (Groups 1 and 2) with those immunized with the fixed cell culture virus vaccine RABIPUR (Group 3) up to six months post-vaccination. A single dose of 10¹⁰ viral particles ChAd155-RG induced the same immune response as three doses of RABIPUR.

These results show that a single administration of ChAd155-RG was able to elicit neutralizing antibody titers well above the seroconversion threshold which are stable over at least 48 weeks and comparable to three doses of RABIPUR. The seroconversion induced by ChAd155-RG was rapid. All animals immunized with ChAd155-RG exceeded the threshold two weeks after immunization, at which time the animals immunized with RABIPUR had already received a second dose.

Boosting the animals in Group 2 with RABIPUR at week 24 (FIG. 18 - squares) was highly effective in raising virus-neutralizing antibodies well above the peak level achieved after the administration of ChAd155-RG at day 0. This demonstrates that the RABIPUR viral lysate antigen is fully able to boost immunity induced by the ChAd155-RG nucleic acid encoded antigen.

Boosting the animals in both Group 1 (FIG. 18 - triangles) and Group 2 (FIG. 18 - upside down triangles) with ChAd155-RG was also highly effective. Animals immunized with ChAd155-RG, regardless of whether or not they were given an intermediate boost with RABIPUR, mounted a robust immune response to the ChAd155-RG boost at week 48. This demonstrates that the ChAd155-RG nucleic acid encoded antigen can be effectively re-administered. It also demonstrates that the ChAd155-RG nucleic acid encoded antigen is effective in boosting the immune response after administration of the RABIPUR viral lysate antigen. In conclusion, Figure 18 demonstrates the compatibility of a simian adenovirus ChAd155 encoding the rabies G antigen with a conventional rabies vaccine comprising a viral lysate antigen.

### Example 9. ChAd155-RG Induces a Cellular Immune Response in Non-human Primates

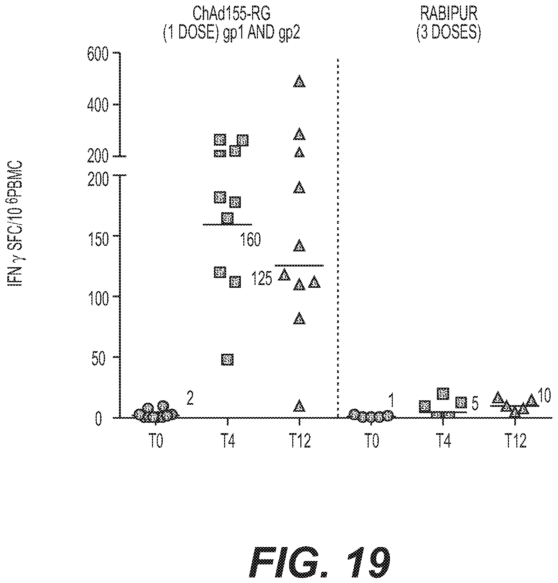

In addition to the humoral antibody response demonstrated in Example 8, ChAd155-RG induced a strong cellular immune response. FIG. 19 shows that a single dose of ChAd155-RG induced a sustained level of rabies glycoprotein specific IFNgammasecreting T-cells in the peripheral blood of vaccinated animals, as detected by IFNgamma-ELlspot assay. In contrast, cellular immune responses were below the limit of detection in the animals vaccinated with RABIPUR.

Animals in Group 1, immunized with ChAd155-RG and boosted with ChAd155-RG at week 48, as described in Example 8, demonstrated that the boost re-amplified IFN gamma levels. Animals in group 2, immunized with ChAd155-RG and boosted first with RABIPUR at week 24 then with ChAd155-RG at week 48 showed no increase in IFN gamma levels in response to the RABIPUR boost but a robust response to the ChAd155-RG boost. This demonstrates that a ChAd155-RG boost can expand memory T cells in mature animals. No interleukin 4 responses were detected over the entire course of the follow up.

### Example 10. Dose Escalation Study for Safety in Humans

To evaluate the safety of ChAd155-RG in humans, a Phase I study will be initiated. Subjects will be normal healthy adult men and women with no history of rabies vaccination, exposure to rabid animals or receipt of an adenovirus-based investigational vaccine. The study size will be large enough to determine the outcome of the primary study endpoint, safety. Standard statistical analyses will be performed, including 95% confidence intervals.

Subjects will receive one or more intramuscular injections of ChAd155-RG; RABIPUR will be used as the comparator. A low dose of the ChAd155-RG vaccine will be administered and, following data review and approval, the dose will then be increased. Subjects will be followed post-administration for systemic and local adverse events, including but not limited to fever, headache, nausea, vomiting, malaise and myalgia; and pain, tenderness, induration, redness or swelling at the injection site. Blood parameters will be examined and any additional unsolicited symptoms will be recorded.

The study may additionally evaluate immunogenicity by assessing vaccine-related immune responses. Outcome measures may include, but not be limited to, levels of serum neutralizing antibodies, quantification of circulating B-cell secreted antibodies and quantification of T-cell responses against a *Lyssaviral* antigen.

## Claims

1. A recombinant adenovirus comprising:
a) a polynucleotide which encodes a polypeptide having the amino acid sequence according to SEQ ID NO: 1 and
b) a polynucleotide which encodes a polypeptide having the amino acid sequence according to SEQ ID NO: 3 and
c) a polynucleotide which encodes a polypeptide having the amino acid sequence according to SEQ ID NO: 5;
wherein the adenovirus comprises a nucleic acid sequence encoding a *Lyssavirus* antigen, wherein the nucleic acid sequence is operatively linked to one or more sequences which direct expression of said *Lyssavirus* antigen in a host cell;
wherein the nucleic acid sequence encoding a *Lyssavirus* antigen encodes an antigen derived from the rabies viral glycoprotein (G).

2. The recombinant adenovirus according to claim 1, wherein the polynucleotide comprises at least one of the following:
a) an adenoviral 5' inverted terminal repeat;
b) an adenoviral EIA region, or a fragment thereof selected from among the E1 A_280R and E1A_243R regions;
c) an adenoviral EIB or IX region, or a fragment thereof selected from among the group consisting of the E1B_19K, E1B_55K or IX regions;
d) an adenoviral E2b region; or a fragment thereof selected from among the group consisting of the E2B_pTP, E2B_Polymerase and E2B_IVa2 regions;
e) an adenoviral L1 region, or a fragment thereof, said fragment encoding an adenoviral protein selected from the group consisting of the L1_13.6k protein, L1_52k and L1_lIIa protein;
f) an adenoviral L2 region, or a fragment thereof, said fragment encoding an adenoviral protein selected from the group consisting of the L2_penton protein, L2_pVll, L2_V, and L2_pX protein;
g) an adenoviral L3 region, or a fragment thereof, said fragment encoding an adenoviral protein selected from the group consisting of the L3_pVl protein, L3_hexon protein and L3_protease;
h) an adenoviral E2A region;
i) an adenoviral L4 region, or a fragment thereof said fragment encoding an adenoviral protein selected from the group consisting of the L4_100k protein, the L4_33k protein and protein L4_VI 11;
j) an adenoviral E3 region, or a fragment thereof selected from the group consisting of E3 ORF1, E3 ORF2, E3 ORF3, E3 ORF4, E3 ORF5, E3 ORF6, E3 ORF7, E3 ORF8, and E3 ORF9;
k) an adenoviral L5 region, or a fragment thereof said fragment encoding the L5_fiber fiber protein;
l) an adenoviral E4 region, or a fragment thereof selected from the group consisting of E4 ORF7, E4 ORF6, E4 ORF4, E4 ORF3, E4 ORF2, and E4 ORF1;
m) an adenoviral 3'-end, preferably an adenoviral 3' inverted terminal repeat; and/or
n) an adenoviral VAI or VAil RNA region, preferably an adenoviral VAI or VAil RNA region from an adenovirus other than ChAd155.

3. The recombinant adenovirus according to claim 1 or 2 wherein:
a) the nucleic acid sequence encoding the Lyssavirus antigen is at least 98.6% identical to SEQ ID NO: 38; and
b) the adenovirus comprises the penton of SEQ ID NO: 3, the hexon of SEQ ID NO: 5 and the fiber of SEQ ID NO: 1 and the Lyssavirus antigen comprises a sequence having at least 90% identity to SEQ ID NO: 37.

4. The recombinant adenovirus according to any one of claims 1 to 3, which is a replication deficient adenovirus.

5. The recombinant adenovirus according to claim 4 wherein the adenovirus comprises:
a) a functional inactivation (such as deletion) of the E1, E3 and/or E4 genes; and/or
b) an Ad5E4orf6 gene substitution.

6. The recombinant adenovirus according to any one of claims 1 to 5, wherein the adenovirus is capable of infecting a mammalian cell.

7. A composition comprising the recombinant adenovirus according to any one of claims 1 to 6 and a pharmaceutically acceptable excipient.

8. The composition according to claim 7, comprising an adjuvant.

9. The recombinant adenovirus of any one of claims 1 to 6 or the composition according to any one of claims 7 to 8 for use in a method of inducing an immune response in a subject, said method comprising administering the adenovirus or composition to the subject.

10. The recombinant adenovirus or composition for use according to claim 9, wherein the subject is infected with a Lyssavirus.

11. The recombinant adenovirus or composition for use according to claim 9 or claim 10, wherein the subject is a human.

## Patentansprüche

1. Rekombinantes Adenovirus, umfassend:
a) ein Polynukleotid, welches ein Polypeptid mit der Aminosäuresequenz gemäß SEQ ID NO: 1 codiert, und
b) ein Polynukleotid, welches ein Polypeptid mit der Aminosäuresequenz gemäß SEQ ID NO: 3 codiert, und
c) ein Polynukleotid, welches ein Polypeptid mit der Aminosäuresequenz gemäß SEQ ID NO: 5 codiert;
wobei das Adenovirus eine Nukleinsäuresequenz umfasst, die ein Lyssavirus-Antigen codiert, wobei die Nukleinsäuresequenz funktionsfähig mit einer oder mehreren Sequenzen verknüpft ist, die die Expression des Lyssavirus-Antigens in einer Wirtszelle steuern;
wobei die ein Lyssavirus-Antigen codierende Nukleinsäuresequenz ein Antigen codiert, welches vom Tollwutvirus-Glycoprotein (G) abgeleitet ist.

2. Rekombinantes Adenovirus gemäß Anspruch 1, wobei das Polynukleotid wenigstens eine/s der folgenden umfasst:
a) eine adenovirale invertierte 5'-terminale Wiederholung;
b) eine adenovirale EIA-Region oder ein Fragment davon, ausgewählt aus den Regionen E1 A_280R und E1A_243R;
c) eine adenovirale EIB- oder IX-Region oder ein Fragment davon, ausgewählt aus der Gruppe, bestehend aus den Regionen E1B_19K, E1B_55K oder IX;
d) eine adenovirale E2b-Region oder ein Fragment davon, ausgewählt aus der Gruppe, bestehend aus den Regionen E2B_pTP, E2B_Polymerase und E2B_IVa2;
e) eine adenovirale L1-Region oder ein Fragment davon, wobei das Fragment ein adenovirales Protein codiert, ausgewählt aus der Gruppe, bestehend aus L1_13.6k-Protein, L1_52k und L1_lIIa-Protein;
f) eine adenovirale L2-Region oder ein Fragment davon, wobei das Fragment ein adenovirales Protein codiert, ausgewählt aus der Gruppe, bestehend aus L2_penton-Protein, L2_pVII, L2_V und L2_pX-Protein;
g) eine adenovirale L3-Region oder ein Fragment davon, wobei das Fragment ein adenovirales Protein codiert, ausgewählt aus der Gruppe, bestehend aus L3_pVI-Protein, L3_hexon-Protein und L3_protease;
h) eine adenovirale E2A-Region;
i) eine adenovirale L4-Region oder ein Fragment davon, wobei das Fragment ein adenovirales Protein codiert, ausgewählt aus der Gruppe, bestehend aus L4_100k-Protein, dem L4_33k-Protein und Protein L4_VI 11;
j) eine adenovirale E3-Region oder ein Fragment davon, ausgewählt aus der Gruppe, bestehend aus E3 ORF1, E3 ORF2, E3 ORF3, E3 ORF4, E3 ORF5, E3 ORF6, E3 ORF7, E3 ORF8 und E3 ORF9;
k) eine adenovirale L5-Region oder ein Fragment davon, wobei das Fragment das L5_fiber-Faserprotein codiert;
l) eine adenovirale E4-Region oder ein Fragment davon, ausgewählt aus der Gruppe, bestehend aus E4 ORF7, E4 ORF6, E4 ORF4, E4 ORF3, E4 ORF2 und E4 ORF1;
m) ein adenovirales 3'-Ende, bevorzugt eine adenovirale invertierte 3'-terminale Wiederholung; und/oder
n) eine adenovirale VAI oder VAII RNA-Region, bevorzugt eine adenovirale VAI oder VAII RNA-Region von einem anderen Adenovirus als ChAd155.

3. Rekombinantes Adenovirus gemäß Anspruch 1 oder 2, wobei:
a) die das Lyssavirus-Antigen codierende Nukleinsäuresequenz zu wenigstens 98,6% identisch zu SEQ ID NO: 38 ist; und
b) das Adenovirus das Penton von SEQ ID NO: 3, das Hexon von SEQ ID NO: 5 und die Faser von SEQ ID NO: 1 umfasst und das Lyssavirus-Antigen eine Sequenz mit wenigstens 90% Identität zu SEQ ID NO: 37 umfasst.

4. Rekombinantes Adenovirus gemäß irgendeinem der Ansprüche 1 bis 3, welches ein replikationsdefizientes Adenovirus ist.

5. Rekombinantes Adenovirus gemäß Anspruch 4, wobei das Adenovirus umfasst:
a) eine funktionelle Inaktivierung (wie beispielsweise Deletion) der E1-, E3- und/oder E4-Gene; und/oder
b) eine Ad5E4orf6-Gensubstitution.

6. Rekombinantes Adenovirus gemäß irgendeinem der Ansprüche 1 bis 5, wobei das Adenovirus zum Infizieren einer Säugerzelle in der Lage ist.

7. Zusammensetzung, umfassend das rekombinante Adenovirus gemäß irgendeinem der Ansprüche 1 bis 6 und einen pharmazeutisch akzeptablen Hilfsstoff.

8. Zusammensetzung gemäß Anspruch 7, welche ein Adjuvans umfasst.

9. Rekombinantes Adenovirus nach irgendeinem der Ansprüche 1 bis 6 oder Zusammensetzung gemäß irgendeinem der Ansprüche 7 bis 8 zur Verwendung in einem Verfahren zum Induzieren einer Immunantwort in einem Subjekt, wobei das Verfahren das Verabreichen des Adenovirus oder der Zusammensetzung an das Subjekt umfasst.

10. Rekombinantes Adenovirus oder Zusammensetzung zur Verwendung gemäß Anspruch 9, wobei das Subjekt mit einem *Lyssavirus* infiziert ist.

11. Rekombinantes Adenovirus oder Zusammensetzung zur Verwendung gemäß Anspruch 9 oder Anspruch 10, wobei das Subjekt ein Mensch ist.

## Revendications

1. Adénovirus recombinant comprenant:
a) un polynucléotide qui code un polypeptide ayant la séquence d'acides aminés selon SEQ ID NO: 1 et
b) un polynucléotide qui code un polypeptide ayant la séquence d'acides aminés selon SEQ ID NO: 3 et
c) un polynucléotide qui code un polypeptide ayant la séquence d'acides aminés selon SEQ ID NO: 5;
où l'adénovirus comprend une séquence d'acide nucléique codant un antigène de *Lyssavirus,* où la séquence d'acide nucléique est liée de manière fonctionnelle à une ou plusieurs séquences qui dirigent l'expression dudit antigène de *Lyssavirus* dans une cellule hôte;
où la séquence d'acide nucléique codant un antigène de *Lyssavirus* code un antigène dérivé de la glycoprotéine (G) du virus rabique.

2. Adénovirus recombinant selon la revendication 1, où le polynucléotide comprend au moins l'un des suivants:
a) une répétition terminale inversée 5' adénovirale;
b) une région EIA adénovirale, ou un fragment de celle-ci choisi parmi les régions E1 A_280R et E1A_243R;
c) une région EIB ou IX adénovirale, ou un fragment de celle-ci choisi dans le groupe consistant en les régions E1B_19K, E1B_55K ou IX;
d) une région E2b adénovirale; ou un fragment de celle-ci choisi dans le groupe consistant en les régions E2B_pTP, E2B_Polymérase et E2B_IVa2;
e) une région L1 adénovirale, ou un fragment de celle-ci, ledit fragment codant une protéine adénovirale choisie dans le groupe consistant en la protéine L1_13.6k, L1_52k et la protéine L1_IIIa;
f) une région L2 adénovirale, ou un fragment de celle-ci, ledit fragment codant une protéine adénovirale choisie dans le groupe consistant en la protéine L2_penton, L2_pVII, L2_V, et la protéine L2_pX;
g) une région L3 adénovirale, ou un fragment de celle-ci, ledit fragment codant une protéine adénovirale choisie dans le groupe consistant en la protéine L3_pVI, la protéine L3_hexon et la L3_protéase;
h) une région E2A adénovirale;
i) une région L4 adénovirale, ou un fragment de celle-ci, ledit fragment codant une protéine adénovirale choisie dans le groupe consistant en la protéine L4_100k, la protéine L4_33k et la protéine L4_VI11;
j) une région E3 adénovirale, ou un fragment de celle-ci choisi dans le groupe consistant en E3 ORF1, E3 ORF2, E3 ORF3, E3 ORF4, E3 ORF5, E3 ORF6, E3 ORF7, E3 ORF8 et E3 ORF9;
k) une région L5 adénovirale, ou un fragment de celle-ci, ledit fragment codant la protéine fibreuse L5_fibre;
l) une région E4 adénovirale, ou un fragment de celle-ci choisi dans le groupe consistant en E4 ORF7, E4 ORF6, E4 ORF4, E4 ORF3, E4 ORF2 et E4 ORF1;
m) une extrémité 3' adénovirale, de préférence une répétition terminale inversée 3' adénovirale; et/ou
n) une région d'ARN VAI ou VAII adénovirale, de préférence une région d'ARN VAI ou VAII adénovirale d'un adénovirus autre que ChAd155.

3. Adénovirus recombinant selon la revendication 1 ou 2, où:
a) la séquence d'acide nucléique codant l'antigène de *Lyssavirus* est identique à au moins 98,6 % à SEQ ID NO: 38; et
b) l'adénovirus comprend le penton de SEQ ID NO: 3, l'hexon de SEQ ID NO: 5 et la fibre de SEQ ID NO: 1 et l'antigène de Lyssavirus comprend une séquence ayant au moins 90 % d'identité avec SEQ ID NO: 37.

4. Adénovirus recombinant selon l'une quelconque des revendications 1 à 3, qui est un adénovirus déficient pour la réplication.

5. Adénovirus recombinant selon la revendication 4, où l'adénovirus comprend:
a) une inactivation fonctionnelle (telle qu'une délétion) des gènes E1, E3 et/ou E4; et/ou
b) une substitution de gène Ad5E4orf6.

6. Adénovirus recombinant selon l'une quelconque des revendications 1 à 5, où l'adénovirus est capable d'infecter une cellule de mammifère.

7. Composition comprenant l'adénovirus recombinant selon l'une quelconque des revendications 1 à 6 et un excipient pharmaceutiquement acceptable.

8. Composition selon la revendication 7, comprenant un adjuvant.

9. Adénovirus recombinant selon l'une quelconque des revendications 1 à 6 ou composition selon l'une quelconque des revendications 7 à 8 pour une utilisation dans un procédé d'induction d'une réponse immunitaire chez un sujet, ledit procédé comprenant l'administration de l'adénovirus ou de la composition au sujet.

10. Adénovirus recombinant ou composition pour une utilisation selon la revendication 9, où le sujet est infecté par un Lyssavirus.

11. Adénovirus recombinant ou composition pour une utilisation selon la revendication 9 ou la revendication 10, où le sujet est un être humain.
